# EUROPEAN PATENT APPLICATION

(11) **EP 4 311 430 A1**
(43) Date of publication of application: **31.01.2024**
(21) Application number: 22306134.2
(22) Date of filing: 28.07.2022
(51) Int. Cl.: A01H 1/00, A01H 5/10, A01H 6/46, C12N 9/02, C12N 15/82

(54) **CHLOROTOLURON TOLERANCE GENE AND METHODS OF USE THEREOF**

(71) Applicant: Limagrain Europe, 63360 Saint-Beauzire (FR)
(72) Inventor: SALLAUD, Christophe, 63720 CHAPPES (FR); ROUSTER, Jacques, 63720 CHAPPES (FR); BIZOUERNE, Elise, 63720 CHAPPES (FR); THROUDE, Mickaël, 63720 CHAPPES (FR); POUPARD, Bruno, 63720 CHAPPES (FR)
(74) Representative: Flesselles, Bruno F.G.

(57) **Abstract**

The present invention relates to the identification of a gene responsible for chlorotoluron tolerance and to methods of uses thereof.

## Description

The present invention relates to the identification of a gene responsible for chlorotoluron tolerance and to recombinant DNA technology using this gene, in particular to the production of transgenic plants which exhibit substantial resistance or substantial tolerance to chlorotoluron.

### BACKGROUND OF THE INVENTION

Chlorotoluron (N'-(3-Chloro-4-methylphenyl)-N,N-dimethylurea), also named chlortoluron, is an organic compound of the phenylurea class of herbicides active on broadleaf and annual grass to control weeds in cereal crops. This compound is described in US2655445. This herbicide acts as an inhibitor of photosynthesis, blocking the Q_{B} plastoquinone binding site of photosystem II, preventing electron flow from Q_{A} to Q_{B}, thus interrupting the photosynthetic electron transport chain and leading to death of the plant. It can be used to control broadleaf weeds and grasses including *Alopecurus myosuroides, Anthemis arvensis, Atriplex prostrata, Calendula spp., Convolvulaceae spp., Galeopsis spp., Lamium spp., Papaver rhoeas, Paspalum distichum, Poa annua, Solanaceae spp., Stellaria media and Veronica spp.* This herbicide is generally applied for removing weeds in cultures of cereals including wheat, barley, rye, triticale, maize, or of potatoes, vegetables including onions, carrot, parsnip, or of fruit including blackcurrant, apples, pears, cherries, strawberry, gooseberry.

Whereas some wheat plants varieties are resistant to this herbicide, other wheats are sensitive. This resistance is likely due to a gene able to detoxify the molecule. A Quantitative Trait Locus (Su1 QTL) has been mapped on the wheat chromosome 6B (Krugman et al., 1997, Theor Appl Genet 94:46-51). This QTL interval corresponds to a region of 450 Mb. This region contains about 10 000 genes, among which multiple genes can be candidate for the phenotype observed (there are multiple cytochromes, that are potentially able to detoxify the herbicide, or Glutathione S-transferases (GST) coding genes, which are also known to be able to detoxify some herbicides). Hence, no gene responsible for such chlorotoluron tolerance has yet been identified.

### SUMMARY OF THE INVENTION

The inventors have identified a gene that is causal for the chlorotoluron tolerance in wheat plants. This gene codes for a cytochrome P450 (CYP71C4). The use of this sequence allows identification of plants that are tolerant to this herbicide by molecular marker genotyping, more accurately than with the markers of the prior art. The gene can also be used as a transgene to induce chlorotoluron resistance in plants that are sensitive to the herbicide. It is also possible to modify expression of the gene in plants, for increasing the resistance (inducing a tolerance to higher amounts or concentrations of chlorotoluron).

In one embodiment, the gene, or a coding sequence (based on the mRNA) thereof, is used as a selectable marker for transforming cells, and in particular plant cells, in transgenesis processes, in particular to positively select cells in which a transgene has been introduced.

In another embodiment, one can use the gene locus as a landing pad to target introduction of transgenes at this specific locus, by restoration (or *de novo* creation) of function of a native defective copy through homologous recombination.

In another embodiment, the gene may be overexpressed (in particular when produced at the UBI (ubiquitin) locus, or by modifying the promoter) to increase chlorotoluron resistance level in organisms (ability to survive or to grow in presence of higher doses of chlorotoluron), in particular plants.

In another embodiment, identification of the gene responsible for chlorotoluron tolerance, and of its natural mutations, makes it possible to develop methods for identifying plants that are naturally susceptible or tolerant to chlorotoluron.

One embodiment also relates to cells, in particular plant cells, and to plants that are transgenic for a functional gene imparting chlorotoluron resistance (coding for SEQ ID NO: 1) and are thus resistant to this molecule. Are also of interest a part, progeny, or asexual propagate of such plants, wherein said part, progeny, or sexual propagate are transgenic for said the functional gene.

Identification of this gene and obtaining transgenic plants makes it possible to perform a method of controlling at least one weed in a field, wherein said field contains at least one transgenic plant expressing SEQ ID NO: 1 as a transgene, comprising applying chlorotoluron to at least a portion of the field.

### DETAILED DESCRIPTION OF THE INVENTION

In a first embodiment, the invention relates to a method for making a cell resistant to chlorotoluron, comprising expressing a protein imparting resistance to chlorotoluron within the cell.

In preferred embodiments, the cell is a plant cell. However, in some embodiments, the cell is a bacterial cell. In other embodiments, the cell is a fungal cell.

In some embodiment, the protein imparting resistance to chlorotoluron is expressed by introduction of a nucleic acid sequence coding for such protein within the cell.

In some embodiment, the nucleic acid sequence is a cDNA sequence, with the non-coding elements (such as promoter, enhancers, terminators, 3'UTR and the like) necessary for expression of the protein.

In other embodiments, the nucleic acid sequence also includes elements such as introns that are excised after transcription.

In some embodiments, the nucleic acid sequence is introduced within the genome of the cell. In other embodiments, the nucleic acid sequence is borne extra chromosomally by a vector, such as a plasmid, a cosmid, a Bacterial Artificial Chromosome, a Yeast Artificial Chromosome.

In some embodiments, the nucleic acid sequence is introduced within the genome of an organelle of the cell (such as the mitochondrial genome or the chloroplast genome). Non-coding regions are thus adapted for expression in these organelles.

In the present application, either term "tolerant" or "resistant" may be used to designate plants or cells that are able to grow in the presence of chlorotoluron when applied at amounts described in the art. In particular, plants resistant to chlorotoluron can grow when the herbicide is applied at an amount of 600 L/ha with the active substance (chlorotoluron) being presence at 3g/L. Such concentration is the one recommended for commercial use of the herbicide. When used *in vitro,* one can use a concentration between about 5 µM and about 150 µM, or between between about 10 µM and about 100 µM, or between about 20 µM and about 100 µM, preferably between about 50 µM and about 100 µM. "Tolerance" or "resistance" refer to the ability to grow in the presence of chlorotoluron.

Either term "sensitive" or "susceptible" may be used in the application to designate plants that are not able to grow in the presence of chlorotoluron that grow slower in the presence of the herbicide. This can be determined by comparing this absence of grow or slower grow in the presence or in the absence of chlorotoluron at concentrations generally used in the art.

It is preferred when the nucleic acid sequence expressing the protein imparting chlorotoluron resistance is introduced within the genome of the cell, and when the cell is a plant.

In particular, the invention relates to a method for making a plant resistant to chlorotoluron, comprising introducing a gene coding for a protein imparting resistance to chlorotoluron in the genome of the cells of the plant.

A protein imparting resistance to chlorotoluron is represented by SEQ ID NO: 1. Such protein may be designated as CYP71C4 in the present application, as it represents a cytochrome P450 family 71 protein. Its cDNA is depicted by SEQ ID NO: 2. The gene coding for such protein is on the chromosome 6B of wheat and SEQ ID NO: 3 represents the two exons, the intron, as well as 5' and 3' UTR SEQ ID NO: 3 corresponds to a sequence that includes elements such as introns that are excised after transcription as mentioned above.

SEQ ID NO: 4 depicts a sequence identified in a plant that is susceptible to chlorotoluron. This sequence corresponds to SEQ ID NO: 2, with the insertion of a "T" nucleotide after nucleotide 787 of SEQ ID NO: 2 (thus being present at position 788 in SEQ ID NO: 4). This insertion introduces a frameshift, and the resulting truncated protein (SEQ ID NO: 5) is thus unable to confer chlorotoluron resistance.

In the context of the present application, a "defective" allele or gene shall designate a nucleic acid sequence that is located at the same locus than SEQ ID NO: 3 on the 6B chromosome of the wheat, and that is not able to product a protein able to detoxify chlorotoluron. This may be done, in particular, by either absence of the gene depicted by SEQ ID NO: 3, presence of a mutation (non-sense, missense, premature stop) in SEQ ID NO: 3, presence of mutations in the promoter of the gene leading to lack of expression of SEQ ID NO: 3... A "functional" allele (as opposed to a defective allele) represents an allele capable to produce SEQ ID NO: 1 or a variant thereof that is capable to detoxify chlorotoluron. Presence of a defective or functional allele may be checked by any method, such as the use of molecular markers, sequencing, or by exposing the cell to chlorotoluron and verifying the growth absence of growth or speed of growth of the cell.

Proteins presenting equal or above 85 %, 86 %, 90%, 92 % or 95%, more preferably equal or above 96%, more preferably equal or above 97%, more preferably equal or above 98%, more preferably equal or above 99% more preferably equal or above 99.5%, more preferably equal or above 99.8% with SEQ ID NO: 1 are variant of SEQ ID NO: 1 and can be considered as equivalent to SEQ ID NO: 1 in the context of the invention, when they impart chlorotoluron resistance or tolerance. Among such variants, which can be allelic versions of SEQ ID NO: 1, the proteins imparting chlorotoluron resistance can be determined by transforming a cell with a nucleic acid sequence encoding said protein and verifying that the cell can grow in presence of chlorotoluron.

Said identity is calculated by using the algorithm of Needleman and Wunsch (J Mol Biol. 1970 Mar;48(3):443-53), using the following parameters: matrix BLOSUM62, gapopen penalty: 10, Gapextension penalty: 0.5. The Needleman and Wunsch algorithm not only provides a percentage of identity between two amino-acid sequences, but also provides a percentage of similarity between said two sequences.

From this protein sequence, critical amino acid localized in the active site can be identified, and such a protein with a lower identity but with conserved amino acid sequence of the critical amino acids of the active site should also fall within the scope of the invention.

Another protein conferring resistance to chlorotoluron is represented by SEQ ID NO: 6, with its cDNA being depicted as SEQ ID NO: 7. The gene coding for such protein is located on chromosome 6 of the barley and is depicted by SEQ ID NO: 8.
SEQ ID NO: 6

MAAQPVLPLLLSTQALLLYLLLIPLLILLRATSRRSAGERAAVDNHGGRPLPSPPNK
LPLIGHLHMVGSDPHVSLAALAAKHGDGGLMLLRLGQTRALVVSSPRAAEAVLRT
QDHVFASRPTNAVVDALLDGGADIAFVPYGEFWRQARRLVTTHLLSARKVHSLR
RGREEEARLVMAKIQGAAAAGATVDMTRLLGAFTNDIVCRAVSGKFFREEGRNE
LFREVIDGNVAAFGGFNLLDYYPGLAKVSMLRRLVFAKTNRLKRKWDDLLDKIIVD
HVSQKASPREQHRDRDQQEDEEETDFVDVLLGLQEEYNLTTEHIKAILMDMFVA
GTDTSYIVLECAMAELMRKPNLMTKLQAEVGEKTPKGQQMVTEDDLGSMAYLKA
VVKETLRLHTPVPLLLPHLSMAECNIDGYTVPAETRVVVNAWALGRDHGSWEAA

GEFMPERFGDIVSPDFKGRDFQFLPFGAGRRMCPGINFAMATVEIMLANLVYCYD
WELPGGMRQEDLDMTDVFGMTMRRKEKLFLVPISRNVNRA

All embodiments herein disclosed with reference to SEQ ID NO: 1 (resp SEQ ID NO: 2; SEQ ID NO: 3) can be similarly performed with SEQ ID NO: 6 (resp SEQ ID NO: 7; SEQ ID NO: 8). For embodiments described with SEQ ID NO: 1 (resp SEQ ID NO: 2; SEQ ID NO: 3) specifically in wheat, the similar embodiments for SEQ ID NO: 6 (resp SEQ ID NO: 7; SEQ ID NO: 8) can be performed in barley.

### MAKING A CELL TOLERANT TO CHLOROTOLURON

A cell can be made resistant or tolerant to chlorotoluron by transformation with a nucleic acid sequence coding for a protein that is able to detoxify this molecule and that is depicted by SEQ ID NO: 1 or SEQ ID NO: 6.

Such transformation is performed by any method known in the art, depending on the nature of the transformation (electroporation, biolistic, *Agrobacterium* transformation for plant cells).

It is to be noted that the method preferably comprises introducing a functional gene in the genome, which would thus be a new gene. This embodiment would be particularly applicable in cells in which there is no gene coding for SEQ ID NO: 1 (or a variant thereof) for instance in maize, or dicotyledonous cells, or in yeast or bacterial cells, but can also be applied in wheat. One can also correct a defective gene present in the genome, in order to obtain a functional copy of the defective gene. This is particularly true for wheat as some lines present an insertion of one or more nucleotide in the coding sequence of the CYP71C4 gene, thereby leading to expression of a non-effective protein.

Thus, in one embodiment, the plant contains defective copies of the CYP71C4 gene coding for the protein imparting resistance to chlorotoluron (for instance a defective copy is represented by SEQ ID NO: 4 in which a "T" nucleotide has been introduced at position 788 (as compared to SEQ ID NO: 3) and coding for a protein that is unable to impart resistance to chlorotoluron and which is represented by SEQ ID NO: 5) and a gene coding for SEQ ID NO: 1 is obtained by gene editing of at least one native defective copies of the CYP71C4 gene in the genome of the plant. In particular, gene editing is performed to delete the "T" nucleotide that is present at position 788 of SEQ ID NO: 4, thereby restoring the correct reading frame for the gene. Gene editing is known in the art and is performed preferably using the CRISPR-Cas9 sequence, so that a deletion (in particular of the extra-"T" nucleotide) is introduced at the proper place within the defective CYP71C4 sequence. When the defectiveness of the gene is due to a point mutation of a nucleotide (being a missense or non-sense, thereby leading to production of a truncated or non-functional protein), base editing is used to replace the point mutation by the proper nucleotide needed to allow production of the functional protein. Using Cytosine and Adenine Base Editors, it is possible to substitute the proper nucleotide to the mutated one. There are multiple scientific publications, patents and applications, describing these processes, using such base editors, (Cas proteins which may be defective in some of their nuclease activities), and template RNAs for introducing the required correction in the genome.

In one embodiment, the gene is corrected by replacing part of the gene, bearing the mutation, by the same part of the gene, but bearing the correction, by homologous recombination. This method is called allele replacement and consists in substituting *in vivo* one allele for another: it is achieved by reciprocal homologous recombination between the sequences of a vector carrying an allele and the chromosome carrying the allele to be replaced (integrative transformation) and makes it possible to replace a defective allele with a functional allele or vice-versa.

One can also envisage the embodiment where the gene coding for SEQ ID NO: 1 is introduced by crossing one plant sensitive to chlorotoluron with a plant bearing the gene coding for SEQ ID NO: 1 (preferably diploid for this gene) and growing a plant selected from the progeny, in which the gene coding for SEQ ID NO: 1 is present. Such plant selection can be performed using adequate markers, or by growing the plant in the presence of chlorotoluron.

One embodiment relates to a method for producing a modified plant having resistance or enhanced tolerance to chlorotoluron, comprising introducing a polynucleotide encoding a chlorotoluron tolerance protein into the cells of a plant to produce said modified plant, wherein the chlorotoluron tolerance protein comprises an amino acid sequence represented by SEQ ID NO: 1, or an equivalent thereof.

In another embodiment, the gene coding for SEQ ID NO: 1 is introduced by transgenesis in the genome of the plant. In particular, it is preferred when the gene is under the control of a promoter that triggers expression at least in the leaves and/or the roots of the plant. Such promoter may be functional only in the leaves, or only in the roots, or both in the leaves and roots. The promoter may also be a constitutive promoter.

The chlorotoluron tolerance protein when expressed in a plant cell renders said plant cell tolerant to chlorotoluron or increases tolerance to this herbicide, as compared to an untransformed plant cell of the same plant. Such enhanced tolerance or resistance to chlorotoluron is observed as compared to the plant without introduction of the gene coding for the chlorotoluron tolerance protein. It is preferred when SEQ ID NO: 1 is expressed at least in the cells of the plant leaves, and preferably in all cells of the plant. The plant may be produced by transforming cells *in vitro,* selecting transformed cells and regenerating a plant from these cells by methods known in the art.

The polynucleotide expresses a chlorotoluron tolerance protein, so that the protein is produced. Consequently, the polynucleotide contains all appropriate elements (such as promoter, enhancer, regulator, terminator...) to allow such protein production within the cell.

One can also cite a method comprising subjecting a plurality of plant cells to transformation with the polynucleotide, growing said cells in a concentration of chlorotoluron that permits transformed cells that express the protein from the polynucleotide to grow while killing or inhibiting the growth of non-transformed cells; and regenerating a plant from said transformed cells.

The invention also relates to a method for performing genetic transformation of cells, comprising
a) Transforming cells with a vector, wherein the vector contains a nucleic sequence encoding SEQ ID NO: 1, wherein the cells are sensitive to chlorotoluron,
b) Growing the transformed cells on a medium containing chlorotoluron
c) Selecting the cells able to grow on the medium of b)

Chlorotoluron is used at a concentration wherein only the cells in which an active SEQ ID NO: 1 is expressed within the cells can grow in or on the medium. The medium can be a solid or a liquid medium. The cells are "sensitive to chlorotoluron" indicates that the cells are not able to grow or to properly be regenerated into a transformed plant or are killed when chlorotoluron is present in the culture medium, absent expression of SEQ ID NO: 1 in the cells.

The vector makes it possible to obtain a genetic modification of a cell, namely the genomic or extragenomic (such as in a plasmid or artificial chromosome) introduction of the nucleic acid encoding SEQ ID NO: 1 in a cell that was devoid of it.

In preferred embodiment, one uses SEQ ID NO: 2 in the nucleic sequence encoding SEQ ID NO: 1. An adequate promoter, as well as other regulatory sequences, would be needed to have the protein expressed, as SEQ ID NO: 2 represents a cDNA. One can also use SEQ ID NO: 7 as the nucleic acid sequence coding for the protein conferring chlorotoluron resistance. In this embodiment, said protein is SEQ ID NO: 6.

Consequently, in one embodiment, the vector contains an expression cassette which comprises a polynucleotide encoding the protein imparting resistance to chlorotoluron; a heterologous promoter that is functional in the cell, and in particular a heterologous promoter functional in a plant cell, said heterologous promoter being operably linked to said polynucleotide. A heterologous promoter is intended to designate a promoter that is not the natural promoter for the gene coding for SEQ ID NO: 1 in wheat (nucleotides 1-444 of SEQ ID NO: 3 provides some natural promoter sequence).

It is reminded that the protein imparting resistance to chlorotoluron is represented by SEQ ID NO: 1, but that some proteins presenting at least 85% sequence identity with SEQ ID NO: 1 have the same effect than SEQ ID NO: 1 with regards to chlorotoluron and can be considered as equivalent to SEQ ID NO: 1.

When expressed in a plant cell, presence of SEQ ID NO: 1 makes the plant cell tolerant or resistant to chlorotoluron, or increases the resistance to this herbicide (for instance by allowing use of higher amounts or concentrations), as compared to an untransformed plant cell of the same plant.

As a heterologous promoter, one uses preferentially a constitutive promoter. Constitutive promoters suitable for use in the present invention include cytomegalovirus (CMV) early immediate gene promoter, simian virus (SV40) promoter, adenovirus major late promoter, Rous sarcoma virus (RSV) promoter, mouse mammary tumor virus (MMTV) promoter, phosphoglycerate kinase (PGK) promoter, EDI-alpha elongation factor promoter, ubiquitin promoters (such as AtUbi10 or AtUbi3 promoters or the ZmUbi promoter (ubiquitin 1 promoter of maize (Christensen et al., 1996, Transgenic. Res., 5: 213)), actin promoters (such as the rice actin (OsAct1) promoter (McElroy et al., 1990, Plant Cell, 2 :163-171)), tubulin promoters, immunoglobulin promoters, alcohol dehydrogenase 1 (ADH1) promoter, RNA polymerase III-dependent promoters such as U6, U3, HI, 7SL, pRPRI (Ribonuclease P RNA 1), SNR52 (small nuclear RNA 52). Among constitutive promoters active in plants, one can cite the CsVMV promoter (Verdaguer et al, 1996, Plant Mol. Biol. 31, 1 129-39 and WO 97/48819), the CaMV 35S (35S promoter of the cauliflower mosaic virus) or the 19S promoter (Kay et al., 1987, Science, 236:1299-1302), the regulatory sequences of the T-DNA of *Agrobacterium tumefaciens,* including mannopine synthase, nopaline synthase, octopine synthase.

As a promoter functional *(i.e.* that is capable of driving expression of the gene) in roots, one can cite IDS2 promoter from barley (Kobayashi et al., 2003) depicted as SEQ ID NO: 9 of WO2017025360, isoflavone synthase gene promoters (IFS1 and IFS2) from soybean (Subramanian et al., Plant Mol Biol. 2004 Mar;54(5):623-39), MsPRP2 promoter from alfalfa (Winicov et al., Planta. 2004 Oct;219(6):925-35), Pyk10, NIP2 and Pht1 promoters from Arabidopsis (Mizutani et al., Plant Cell Physiol. 2006 Oct;47(10):1420-6; Mudge et al., Plant J. 2002 Aug;31(3):341-53; Nitz et al., Plant Sci. 2001 Jul;161(2):337-346), several root-specific promoters such as tobacco TobRB7, strawberry FaRB7, tomato LcRB7, western white pine PsPR10 and PmPR10, and others have been isolated (Nan et al. 2002 (X. Xu et al. Biotechnol Lett. 2010 Oct;32(10):1533-9; Liu and Ekramoddoullah Plant Mol Biol. 2003 May;52(1):103-20; Vaughan et al. J Exp Bot. 2006;57(14):3901-10). maize ZmTIP2-3, depicted as SEQ ID NO: 11 of WO2017025360, GmTIP (Chen et al. Plant Cell, Tissue and Organ Culture (PCTOC) volume 121, pages 259-274 2015), GmPRP1 and GmPRP2 exhibit root-specific expression (Suzuki et al., Plant Mol Biol. 1993 Jan;21(1):109-19; Hong et al., Plant Cell. 1989 Sep;1(9):937-43. doi: 10.1105/tpc. 1.9.937), RSP1, rRSP3, and rRSP5, Os03g01700 depicted as SEQ ID NO: 12 of WO2017025360 and Os02g37190, proOsRCG2 is a promoter from Oryza sativa (rice) highly expressed only in root tissues according to (Y. Xu et al. 1995) and depicted as SEQ ID NO: 10 of WO2017025360, pR110 promoter, also designated as RCc3 promoter (Plant Mol Biol. 1995 Jan;27(2):237-48) and depicted as SEQ ID NO: 13 of WO2017025360, Medicago phosphate transporter: Xiao et al.., 2008, Plant Biol (Stuttg). 2008 Jul:8(4)439-49, tobacco auxin-inducible gene: Van der Zaal et al Plant Mol Biol. 16, 983, 1991, tobacco root-specific genes: Conkling, et al, Plant Physiol. 93, 1203: 1990, B. napus G1-3b gene: US 5,401,836, SbPRP1: Suzuki et al., Plant Mol, Biol. 21: 109-119, 1993, LRX1: Baumberger et al, 2001, Genes & Dev, 15;1128, *B. napus* BTG-26 Brassica US 20050044585, class I patatin gene (potato): Liu et al" Plant Mol, Biol. 17 (6): 1139-1154, or ALFS *(Arabidopsis):* Diener et al. (2001, Plant Cell 13:1625).

As leaf specific promoters, one can cite the ones described in US9163251B2, the Zea *mays* Zmglp1, *Pharbitis nil* PnGLP, *Oryza sativa* PDX1, the B. *distachyon* promoters described by Alotaibi (Saudi Journal of Biological Sciences, 28(9) 2021, 5187-5192), promoters described in Alotaibi et al (Plants (Basel). 2018 Jun; 7(2): 27), promoters of Xun et al (Transgenic Res 30, 799-810 2021)

As termination sequences, one can cite the 3' *Agrobacterium tumefaciens* Nopaline synthase (Nos) termination sequence (terAtNos) or the *Arabidopsis thaliana* Sac66 termination sequence (terAtSac66).

In some embodiments, the vector also comprises at least one other sequence of interest coding for another protein imparting a phenotypical trait of interest. Such other transgene can be a gene providing a trait selected from the group consisting of insect resistance, herbicide resistance, fungal resistance, stress tolerance, increased yield, improved oil profile, improved fiber quality, viral resistance, delayed ripening, cold tolerance, and salt tolerance. Such transgenes of agronomic interest are known in the art.

In particular, an insect-resistance gene can be derived from an organism selected from the group consisting of *Bacillus thuringiensis, Photorhabdus,* and *Xenorhabdus.* Herbicide resistance genes include genes imparting resistance to glyphosate, glufosinate, dicamba, acetolactate synthase (ALS) inhibitors, protoporphyrinogen oxidase (PPO) inhibitors ACCAse inhibitors (herbicides belonging to Aryloxyphenoxypropionate (FOPs), cyclohexanedione (DIMs), and phenylpyrazolin (DENs) chemistries), and 4-hydroxyphenyl-pyruvate-dioxygenase (HPPD) inhibitors.

In one embodiment, the genetic cassette containing the gene coding for the CYP71C4 protein, and optionally at least one other gene of interest, is introduced by homologous recombination within the cell genome. In this embodiment, a cassette containing the CYP71C4 gene and optionally one of more transgene(s) is flanked by two recognition sequences that are homologous or identical to two sequences present at a given locus of the genome of the plant. Preferably, the nucleic acid bearing the cassette and the recognition sequences is linear. A double strand break is performed in the vicinity of this locus, using nucleases as disclosed in WO 2007/135022 or a CRISPR/Cas endonuclease, using a gRNA that recognizes a sequence that is in the vicinity of the locus to target the nuclease at this site, and induce the double strand break.

In a specific embodiment, the locus for homologous recombination is the CYP71C4 locus. It is reminded that the sequence of the CYP71C4 locus is present in sequence NC_057810.1 in the NCBI DNA sequence database. SEQ ID NO: 3 corresponds essentially to the sequence extending from nucleotide 166925876 to 166928875 of NC_057810.1. It is to be noted that NC_057810.1, obtained from Chinese Spring, contains the "T" insertion that is present at position 788 in SEQ ID NO: 4. In view of this insertion, the sequence provided in NC_057810.1 is a sequence obtained from a plant that is susceptible to chlorotoluron. Some minor nucleotide substitutions are also present between SEQ ID NO: 3 and NC_057810.1; However, using this referenced sequence recorded in a database, one of skill in the art is able to retrieve sequences that are located 5' and 3' of SEQ ID NO: 3, and to determine the chromosomal locus of CYP71C4 on the 6B chromosome of a wheat plant.

In this embodiment, it is preferred when the cell contains defective copies of the CYP71C4 gene, and when the homologous recombination vector is designed so that a functional version of the CYP71C4 gene is reconstituted upon homologous recombination.

### LANDING PAD

When producing transgenics events, the transgenes are usually randomly integrated into the plant genome. This procedure is not optimal for several reasons: there is a risk of integration of the transgene into endogenous genes, potentially causing a loss of function or altered functionality of these endogenous genes, or of creation of novel open reading frames. The site of insertion and of the surrounding genomic environment at this site can influence the level of expression of the transgene. It is also possible to obtain multiple insertions of the transgenes, which is generally not sought. Positioning of a transgene at a defined place in the genome (named a Landing Pad) addresses the concerns outlined above.

One can use the CYP71C4 locus as a landing pad, i.e. a favored locus to integrate transgenes.

In this embodiment, one shall use homologous recombination to replace a non-functional version of the CYP71C4 gene (in particular the one that includes SEQ ID NO: 4) by the functional version, as depicted by SEQ ID NO: 3.

The principle is to provide, in a vector, an incomplete version of the gene coding for SEQ ID NO: 1, wherein the functional version of the gene (presence of SEQ ID NO: 3) is reconstituted upon homologous recombination. Using homologous recombination sequences, part of the CYP71C4 gene containing the mutation responsible for the defective nature of the gene/protein is excised and replaced by the corresponding part without the mutation. While correcting the gene, it is also possible to introduce (upstream (in 5' or downstream (in 3')) a transgene at the location. This method allows to specifically target the site of integration of the transgene. Furthermore, this makes it possible to select the cells using chlorotoluron tolerance as the selection marker, while ensuring that the expression cassette has been integrated at the proper location.

This principle is similar to the allele replacement method for providing chlorotoluron resistance to a cell, but differs in that, in the allele replacement method, there is no transgene integration (homologous recombination is only used to substitute a mutated part of the CYP71C4 gene by the non-mutated part).

In this embodiment, homologous recombination is performed by providing a vector (or cassette) preferably linearized comprising the transgene(s) to integrate within the cell genomes, which is flanked by two recognition regions (Y and Y') that are homologous or identical to genomic regions of the CYP71C4 locus, and which will serve as templates to initiate the recombination event. Preferably, the genome of the plant is cut near one or both recognition regions, as homologous recombination is favored when nucleic acid presents "open" or "cut-off" ends (i.e. is linear).

The Y and Y' sequences are said to be "homologous" if they present at least 90%, more preferably at least 95%, even more preferably 100% identity, over their length with the genomic sequence. It is indeed preferred to have strict identity in order to improve the targeting of the region where homologous recombination is to be performed.

The inventors have shown that, most of the time, the non-functional version of the gene is due to the presence of a "T" nucleotide at position 788 of SEQ ID NO: 3, thereby introducing a frameshift. The method herein disclosed is of particular interest in plants sensitive to chlorotoluron, as the integration of the cassette by homologous recombination will reconstitute a functional gene, thereby allowing screening of plants having undergone homologous recombination (and this integration of the transgene) by using chlorotoluron as a selection marker.

In this embodiment, and to be able to reconstitute a functional gene, one recognition site consists in part of the CYP71C4 gene, and contains the GGACCACG sequence (nucleotides 783-790 of SEQ ID NO: 3) (i.e. the sequence of the CYP71C4 gene without the "T" insertion).

### When the inserted transgene is introduced 5' from the CYP71C4 gene.

In this embodiment, the cassette is designed so as to replace a genomic region located within 5' of the CYP71C4 gene. The transgene is introduced upstream (in 5') of the CYP71C4 gene (preferably upstream the CYP71C4 promoter, unless an operon system is envisaged).

In one embodiment, the cassette may contain (from 5' to 3')
- A recognition region Y located at its 5' end (5' recognition region or 5' homologous region) that comprises at least 200 nucleotides, more preferably at least 300 nucleotides, more preferably at least 400 nucleotides, more preferably at least 500 nucleotides, more preferably at least 600 nucleotides located within the genome 5' from nucleotide 1 of SEQ ID NO: 3. In some embodiments, the last nucleotide of the 5' recognition site being located about 500-1000 bases 5' from nucleotide 1 of SEQ ID NO 3. Use of the sequence of NC_057810.1 makes it possible to determine the sequence to use in the 5' recognition site.
- The transgene (gene of interest, GOI) to be inserted within the genome, comprising the elements allowing transcription of the transgene and expression of the protein that it encodes.
- A sequence P containing a promoter to drive expression of the CYP71C4 gene (preferably comprising 1-444 of SEQ ID NO: 3)
- A sequence E1 containing at least nucleotide 445-790 of SEQ ID NO: 3, thus containing the start of the ORF of the CP71 gene, including the GGACCACG sequence,
- A recognition region Y' located at its 3' end (3' recognition region or 3' homologous region) that contains at least 200 nucleotides, more preferably at least 300 nucleotides, more preferably at least 400 nucleotides, more preferably at least 500 nucleotides, more preferably at least 600 nucleotides of SEQ ID NO: 3, located just after the sequence E1 in SEQ ID NO: 3 (3' recognition region thus contains part of SEQ ID NO: 3 located after the GGACCACG sequence).
The 3' recognition region shall not contain the whole coding sequence of the CYP71C4gene (i.e. it should not extend up to nucleotide 2134 of SEQ ID NO: 3). This is because the end of the CYP71C4 gene sequence in the chromosome after homologous recombination shall originate from the original chromosome.

One can also say that the 3' recognition region contains nucleotides 1-790 of SEQ ID NO: 3.

Then the chromosome is cut-off for favoring homologous recombination, this embodiment can be performed by opening the CYP71C4 gene (for instance using the CRISP-Cas9 system with 2 gRNAs (guide RNAs),
- One gRNA targeting in the genomic region upstream of the CYP71C4 gene promoter (upstream nucleotide 1 of SEQ ID NO: 3), and downstream the 5' recognition sequence (cleavage site 1 region) and
- the other gRNA targeting a sequence downstream of the GGACCTACG sequence in order to remove the non-functional version of the CYP71C4 gene, and upstream the 3' recognition region (cleavage site 2 region).

This will thus delete part of the CYP71C4 gene.

In order to insert the GOI expression cassette and to reconstitute a functional CYP71C4 gene, the fragment to be inserted within the deleted CYP71C4 locus is as described above. The 3' recombination region contains genomic sequence upstream of the cleavage site 1 region and the 5' recombination region contains genomic sequence downstream of the cleavage site 2 region (downstream of the GGACCTACG sequence).

In order to avoid further cleavage of the repair fragment containing the CYP71C4 fragment with the corrected GGACCACG sequence and the GOI expression cassette, the 2 gRNA recognition sites, in the cassette, are preferably mutated in such a way that there is no recognition of repair fragment by the gRNA used for inducing partial deletion of the CYP71C4 locus. Mutation is chosen in order to be further transcribed into the same amino-acid sequence,.

Upon homologous recombination, the 5' and 3' recognition sequences are introduced within the cell genome (together with the cassette) and replace the sequence naturally present within the genome. Hence, upon homologous recombination, the GGACCACG is introduced within the chromosome and replaces the GGACCTACG sequence. If homologous recombination has occurred, the reading frame for the CYP71C4 gene is conserved. Consequently, the gene that will be present in the cell chromosome after homologous recombination will include the GGACCACG sequence rather than the GGACCTACG sequence and will produce a protein that will confer chlorotoluron resistance. Chlorotoluron can thus be used as the selection marker to select cells in which homologous recombination has occurred.

*When the inserted transgene is introduced downstream (3') from the* CYP71C4 *gene.*

In this embodiment, the cassette is designed so as to replace a genomic region located within the 3' end of the CYP71C4 gene. The transgene is introduced downstream (in 3') of the CYP71C4 gene.

In one embodiment, the cassette may contain (from 5' to 3')
- A recognition region Y located at its 5' end (5' recognition region or 5' homologous region) that comprises at least 200 nucleotides, more preferably at least 300 nucleotides, more preferably at least 400 nucleotides, more preferably at least 500 nucleotides, more preferably at least 600 nucleotides located within the genome 5' and including the sequence GGACCACG (nucleotides 782-790 of SEQ ID NO: 3). Preferably, the 5' recognition site should not contain all nucleotides between 1 and 790 of SEQ ID NO: 3. In some embodiments, the 5' recognition site doesn't contain all nucleotides between 445 and 790 of SEQ ID NO: 3. This is to avoid presence of the complete CYP71C4 gene in the cassette and allow detection of integration by homologous recombination by using the CYP71C4 protein as a selection marker.
- A sequence E2 comprising nucleotides of SEQ ID NO: 3 from the last nucleotide of Y, and extending up to nucleotide 2134 of SEQ ID NO: 3, thus containing the CYP71C4 open reading frame.
- The transgene(s) (gene of interest, GOI) to be inserted within the genome, comprising the elements allowing transcription of the transgene and expression of the protein that it encodes.
- A recognition region Y' located at its 3' end (3' recognition region or 3' homologous region) that contains at least 200 nucleotides, more preferably at least 300 nucleotides, more preferably at least 400 nucleotides, more preferably at least 500 nucleotides, more preferably at least 600 nucleotides, located just after nucleotide 2200 in SEQ ID NO: 3 (the 3' recognition region may thus contains part of SEQ ID NO: 3, or genomic sequences not specified in SEQ ID NO: 3 but that may be determined using NC_057810.1).
The 3' recognition region will recognize a genomic region locate downstream in 3' of the CYP71C4 encoding gene.

When the chromosome is cut for favoring homologous recombination, this embodiment can be performed by opening the CYP71C4 gene (for instance using the CRISP-Cas9 system with 2 gRNAs (guide RNAs),
- One gRNA targeting a sequence in the genomic region preferably upstream of the GGACCTACG sequence and downstream the 5' recognition sequence (cleavage site 1 region) and
- the other gRNA targeting a sequence downstream of the GGACCTACG sequence and upstream the 3' recognition region (cleavage site 2 region).

This will thus partially delete the genomic CYP71C4 locus.

In order to insert the GOI expression cassette and to reconstitute a functional CYP71 gene, the fragment to be inserted within the partially deleted CYP71C4 locus is as described above. The 3' recombination region contains genomic sequence upstream of the cleavage site 1 region and the 5' recombination region contains genomic sequence downstream of the cleavage site 2 region (downstream of the GGACCTACG sequence).

In order to avoid further cleavage of the repair fragment containing the CYP fragment with the corrected GGACCACG sequence and the GOI expression cassette, the 2 gRNA recognition sites, in the cassette, are preferably mutated in such a way that there is no recognition by the gRNA used for inducing partial deletion of the CYP71 locus, Mutation is chosen in order to be further transcribed into the same amino-acid sequence.,

Hence, upon homologous recombination, the 5' and 3' recognition sequences are introduced within the genome as they are substituted to the homologous sequences present in the genome. The GGACCACG sequence and the sequence 5' from this sequence are introduced within the chromosome and will replace the GGACCTACG sequence and genomic 5' sequences to generate a functional CYP71 gene thereof. If homologous recombination has occurred, this warrants that the frame is conserved. Consequently, the gene that will be present in the cell chromosome after homologous recombination will include the GGACCACG sequence rather than the GGACCTACG sequence and will produce a protein that will confer chlorotoluron resistance. Chlorotoluron can thus be used as the selection marker to select cells in which homologous recombination has occurred.

The transgenes introduced with these methods may be in the same or in the opposite direction as the CYP71C4 encoding gene.

If the non-functional gene presents another mutation than the introduction of the "T" after position 787 of SEQ ID NO: 3, the recognition sites shall be adapted.

As indicated above, the principle of using the CYP71C4 locus as a landing pad is to replace the part of the CYP71C4 gene by a functional part. As a result, chlorotoluron can be used as the selection marker to select cells in which homologous recombination has occurred, and which are thus able to grow in presence of chlorotoluron. These cells will thus also have integrated to the other sequence(s) of interest.

As indicated above, it is known that creation of a double strand break (DSB) or breaks in the vicinity of the site in which homologous recombination is wished will increase the frequency of HR. These DSB may be created by the use of a restriction enzyme, or a meganuclease, (a specific restriction enzyme that recognizes and cut DNA at long (more than 15 bp) sites) or preferably by use of the CRISPR/Cas system.

One can use a CRISPR-Cas endonuclease expression cassette that comprises a nucleic acid encoding a Cas endonuclease under the control of a promoter, and preferably a terminator. The promoter can be any promoter usable in the cell, in particular a constitutive promoter. Such promoters are disclosed above, and include the ZmUbi promoter, the 35S promoter, the 19S promoter, the rice actin promoter, the pCRV promoter, the CsVMV promoter and any other ubiquitin promoter in particular from a cereal, or from *Arabidopsis.*

The Cas endonuclease is an enzyme which uses a gRNA as a guide to recognize a specific position in a DNA sequence, the protospacer, and performs a double-stranded break. The Cas endonuclease generally requires the presence of a Protospacer Adjacent Motif (PAM) sequence in the vicinity of the specific targeted position, which PAM sequence can differ depending on the Cas endonuclease.

The Cas endonuclease may be selected from the group consisting of Cas9, Cas12a, Cas12b, C2c1 and C2c2, and is preferably the Cas9 (CRISPR-associated protein 9) endonuclease.

The gRNA shall be selected to target a sequence in the vicinity (within 50-150 bases) of one recognition sequence, thus inducing the double strand break and improving homologous recombination.

### GENE EDITING

In some embodiments, a functional gene coding for the protein conferring chlorotoluron resistance is introduced by editing a non-functional gene.

In particular, this method is used to delete the "T" nucleotide that is seen in the CYP71C4 gene of plants susceptible to chlorotoluron, and that leads to production of a non-functional protein.

Gene editing (GE) by the CRISPR/Cas system is a tool widely used worldwide to edit prokaryotic and eukaryotic genomes (Jinek, M. et al. Science 337, 816-821 2012, Cong, L. et al. Science 339, 819-823 2013).

Various types of editing can be obtained after a Double Stranded-Break (DSB) made by a CRISPR/Cas system. "SDN1" editing means that the repair of the DNA strands is made by one pathway of the cellular DNA repair machinery: the Non-Homologous End Joining pathway (NHEJ). The repair can result in small insertions or deletions (indels) at the cleavage site. "SDN2" is an editing where the repair is made by the insertion of a sequence by homologous recombination (HR) using a template. The insertion is usually identical to the target sequence but comprises a desired correction.

Both "SDN1" and "SDN2" types can be used to delete the inserted "T" nucleotide. In case the gene is absent or bears another mutation, the choice of the type of the editing system can be adapted.

One can also use the prime Editing technology, originally described in animal cells by Liu (D. Liu. CRISPR Meeting CSHL 2019) and Anzalone et al. 2019. This technology uses a Reverse Transcriptase (RT) fused with a CRISPR Cas9 nickase to obtain editing in their target cells, programmed with a prime editing guide RNA (pegRNA) that both specifies the target site and encodes the desired edit. WO2021165508 discloses specific RT proteins that can be used for performing this method.

In particular, one can perform an edit within the CYP71C4 gene (in particular deleting the "T" nucleotide present at position 788 of SEQ ID NO: 4), by:
a. providing a Cas nickase associated with a reverse transcriptase, preferably adapted to plants, and a prime-editing guide RNA (pegRNA) to the double-stranded DNA sequence, wherein the pegRNA comprises (from 5' to 3'):
   i) a single guide RNA region which hybridizes to a DNA strand at the target site (here the CYP71C4 gene site at which the edit is to be made), thereby directing the Cas nickase associated with the reverse transcriptase to the site,
   ii) a guide scaffold sequence which allows Cas-binding,
   iii) a template RNA containing the desired edit (in particular deletion of the T nucleotide, and which will serve as the template for creating the edited DNA strand, and
   iv) a primer binding site (PBS) that allows the 3'end of the nicked DNA strand to hybridize to the pegRNA, and will serve for the reverse transcriptase to start reverse transcription;
b. wherein one strand of the double-stranded DNA sequence is cut by the nickase thereby generating a free single-strand DNA (ssDNA) having a 3' end;
c. wherein the 3' end of the free ssDNA hybridizes to the PBS of the pegRNA,
d. wherein the reverse transcriptases performs reverse transcription of the template RNA of the pegRNA and elongates the 3' extremity of the ssDNA,
e. thereby generating a ssDNA flap comprising the desired edit and which is complementary to the DNA synthesis template.
f. wherein the endogenous DNA strand adjacent to the cut site is replaced with the ssDNA flap, thereby installing the desired edit at the target site in the double-stranded DNA sequence.

### DNA SHUFFLING

It is possible to obtain variants of the nucleotide and amino acid sequences herein disclosed by mutagenic and recombinogenic procedures, such as DNA shuffling.

With such a procedure, it is possible to create a new protein that is resistant to chlorotoluron and imparting another phenotype (such as insect resistance or resistance to another herbicide), by using one or more different protein coding regions can be used to create a new protein possessing the desired properties (resistance to chlorotoluron and other trait). To perform such method, libraries of polynucleotides are generated, using polynucleotides which present substantial sequence identity, so as to allow homologous recombination *in vitro* or *in vivo.*

Using this method, the regions encoding the domain imparting chlorotoluron resistance of the CYP91C4 gene may be shuffled with other known domains of interest of other genes to obtain a new gene coding for a protein with an improved property. Homologous regions can be introduced in the domains to allw efficient recombination. Strategies for DNA shuffling are known in the art, and are described, in particular in Stemmer (1994, Proc. Natl. Acad. Sci. USA 91:10747-10751), Stemmer (1994, Nature 370:389-391), Crameri et al. (1997, Nature Biotech. 15:436-438), Moore et al. (1997, J. Mol. Biol. 272:336-347), Zhang et al. (1997, Proc. Natl. Acad. Sci. USA 94:4504-4509), Crameri et al. (1998, Nature 391:288-291), or in US 5,605,793 or US 5,837,458.

### INTRODUCTION AT THE UBI LOCUS

In one embodiment, the gene encoding the protein conferring chlorotoluron resistance is introduced at the UBI (ubiquitin) locus. It is indeed known that fusion proteins consisting of ubiquitin linked to the N terminus of a protein of interest are efficiently processed by ubiquitin-specific proteases into their respective free proteins within eukaryotic cells (Bachmair et al., Science. 1986;234(4773):179-86 ; Varshavsky, Methods Enzymol. 2005; 399:777-99; US7087811). The invention thus also relates to a method for making a transgenic plant, comprising introducing a nucleic acid sequence coding for the protein imparting chlorotoluron resistance (SEQ ID NO: 1) at the UBI locus of a plant, wherein such nucleic acid sequence is in frame with the UBI gene, so that a fusion protein UBI-SEQ ID NO: 1 or SEQ ID NO: 1-UBI is produced. The transformation is generally performed in plant cells, and a plant is regenerated from the cells in which the gene coding for SEQ NO: 1 has been inserted at the UBI locus in such frame that a fusion protein is obtained.

In order to proceed, one can use the CRISPR/Cas system, known in the art to induce cutting at the Ubi locus, and introduce the nucleic acid sequence encoding SEQ ID NO: 1 by homologous recombination (HR). Since SEQ ID NO: 1 confer chlorotoluron resistance, plants in which HR has occurred can easily be identified by their ability to grow or not die in presence of this herbicide.

One shall thus use a vector suitable for a targeted integration of at least one gene of interest in 5' or 3' of a polyubiquitin gene in a plant, wherein said vector comprises a repair DNA comprising 5 from 5' to 3':
- a first gRNA target,
- a left ubiquitin-like region,
- a sequence coding for SEQ ID NO: 1 (in particular SEQ ID NO: 2),
- a right ubiquitin-like region, and
- a second gRNA target.

The vector may also comprise:
- at least one CRISPR-Cas endonuclease expression cassette and/or
- at least one gRNA expression cassette encoding a gRNA able to recognize a region in 3' or 5' of the polyubiquitin gene.

Alternatively, the at least one CRISPR-Cas endonuclease expression cassette and/or the at least one gRNA expression cassette may be provided in the form of one or several separate vector(s) comprising said cassettes.

The gRNA expression cassette comprises a nucleic acid encoding a gRNA under the control of a promoter and comprises:
- a region complementary to the first and/or second gRNA target sequence and/or to a region in 5' or 3' of the polyubiquitin gene, and
- a scaffold region that allows binding to the CRISPR-Cas endonuclease encoded by the CRISPR-Cas endonuclease expression cassette.

The first gRNA target and the second gRNA target comprise a sequence complementary to a gRNA. In one embodiment, the first gRNA target and the second gRNA target may comprise a sequence complementary to the same gRNA.

The gRNA target preferably comprises at least 15 nucleotides, preferably at least 17 nucleotides, more preferably at least 18 nucleotides and/or at most 25 nucleotides, preferably at most 22 nucleotides, more preferably at most 20 nucleotides. The gRNA target for example consists of 17, 18, 19 or 20 nucleotides. The first and second gRNA target sequences are preferably identical.

An ubiquitin-like region preferably comprises:
- a sequence homologous to a sequence comprising the end of the coding region of the polyubiquitin gene and at least a portion of the 3'UTR region of the polyubiquitin gene, for a targeted insertion in 3' of the polyubiquitin gene, or
- a sequence homologous to a sequence comprising at least a portion of the 5'UTR region of the polyubiquitin and the start of the coding region of the polyubiquitin gene, for a targeted insertion in 5' of the polyubiquitin gene.

The ubiquitin-like region as defined above comprises a left ubiquitin-like region in 5' and a right ubiquitin-like region in 3'. Such an ubiquitin-like region may thus comprise, between the left ubiquitin-like region and the right ubiquitin-like region, a sequence homologous to a sequence of the polyubiquitin gene, which is lost upon the targeted integration of the at least one gene of interest. Alternatively, the ubiquitin-like region may consist of a left ubiquitin-like region in 5' and a right ubiquitin-like region in 3'.

A left ubiquitin-like region, in particular suitable for a 3' insertion, may for example comprise or consist of a sequence homologous to repeats 3 to 5 of the polyubiquitin gene Ubi7DL. A right ubiquitin-like region, in particular suitable for a 5' insertion, may for example comprise or consist in a sequence homologous to repeats 1 to 3 of the polyubiquitin gene Ubi7DL. A right ubiquitin-like region, in particular suitable for a 3' insertion, may comprise or consist of a sequence homologous to a region of the 3'UTR region of the polyubiquitin gene Ubi7DL. A left ubiquitin-like region, in particular suitable for a 5' insertion, may comprise or consist of a sequence homologous to a region of the 5'UTR region of the polyubiquitin gene Ubi7DL.

The left and/or right ubiquitin-like region preferably comprises at least 200 nucleotides, more preferably at least 300 nucleotides, more preferably at least 400 nucleotides, more preferably at least 500 nucleotides, more preferably at least 600 nucleotides and/or at most 1900 nucleotides, preferably at most 1700 nucleotides, more preferably at most 1500 nucleotides, more preferably at most 1300 nucleotides, more preferably at most 1100 nucleotides, more preferably at most 900 nucleotides.

When the integration occurs at the start codon, the start codon of the gene of interest replaces the start codon of the polyubiquitin gene. In this embodiment, the repair DNA for example comprises from 5' to 3':
- a first gRNA target,
- a left ubiquitin-like region,
- a sequence coding for SEQ ID NO: 1,
- a sequence coding for a site cleavable by a Ubi protease,
- the start codon of the first Ubi repeat of the polyubiquitin gene,
- a right ubiquitin-like region, and
- a second gRNA target.

In another embodiment, integration of the sequence coding for SEQ ID NO: 1 occurs 3' of the polyubiquitin gene, and the construct is made so that the polyubiquitin stop codon is replaced by the first codon of the sequence coding for SEQ ID NO: 1.

### MODIFICATION OF THE PROMOTER

In one embodiment, the invention pertains to a method for increasing resistance to chlorotoluron in a cell or in a plant, comprising modifying the sequence of the promoter of the gene coding for SEQ ID NO: 1 so that expression of SEQ ID NO: 1 is increased (such increase in expression can be verified, in particular by Northern or Western blot).

Modification of the promoter sequence (which is represented in particular by nucleotides 1-444 of SEQ ID NO: 1) can be performed by adding, modifying or deleting Cis regulatory Elements (CRE) in the promoter region of CYP71C4. This may be done by homologous recombination or gene editing.

This allows obtaining plants or cells in which expression of CYP71C4 is enhanced as compared to a plant in which the CRE have not been modified. Thus, the plant or the cell is resistant to higher amounts/concentrations of chlorotoluron (is able to grow in presence of a concentration of amount of chlorotoluron for which the non-modified plant or cell is not able to grow).

One can cite in particular Schmitz et al (Plant Cell. 2022 Feb; 34(2): 718-741) or Pandiarajan and Grover (Plant Sci. 2018 Dec; 277: 132-138) who describe modifications of promoters.

In one embodiment, suppression of silencer sequences (also known as repressors) in the promoter region of CYP71C4 encoded by SEQ ID NO: 2 by deletion of the silencer region using CRISPR/Cas9 can lead to an increase of the CYP71C4 gene expression

In one embodiment, increase of the CYP71C4 gene expression can be achieved by Targeted Promoter Polymorphism (TPP) using the multiplexing capabilities of the CRISPR/Cas9. By targeting multiple gRNAs towards sequences of fixed distance from TSS of a gene, one can generate variations in the promoter sequences in offspring and select for plants with alterations in the desired trait. Once the plant with desired results is identified, the promoter modification can then be fixed by segregating out the CRISPR/ Cas9 cassette integrated within the genome. This technique has been recently experimented in three genes in tomato [Rodriguez-Leal et al., Cell. 2017 Oct 5;171(2):470-480.e8].

In one embodiment, insertion of enhancer elements such as TE or MITE sequences (Hpscotch1 for tb1 maize promoter (see Studer et al, Nat Genet. 2011 Sep 25;43(11):1160-3) or MITE and solo-LRT for Abp1 promoter) upstream of the ATG of the CYP71C4 gene by homologous recombination increase, similarly to the method described by Elrouby and Bureau (Molecular Genetics and Genomics volume 287, pages 143-153 2012) for modulation of AUXIN-BINDING PROTEIN 1 gene expression in maize and the teosintes by transposon insertions in its promoter.

In another embodiment, the CYP71C4 coding sequence is brought in the vicinity of the promoter of a highly expressed gene following the deletion of the region between the promoter of the highly expressed gene and the coding region of the CYP71C4 gene. This corresponds to the promoter stitching approach described in the patent application EP21305141.0. The gene providing its promoter region fused to the CYP71C4 coding region can be any highly expressed gene in the same orientation and located upstream of the CYP71 gene as for instance the fatty acid oxidase (TraesCS6B02G153900) gene.

The same methods can be used to reduce the expression of the promoter and so to increase susceptibility of the plant to the herbicide. Some uses of susceptible plant are described in this application, notably in method for hybrid production.

### TRANSFORMATION METHODS / VECTORS

The invention also encompasses a vector containing the nucleic acid construct (expression cassette) containing a nucleic acid sequence coding for SEQ ID NO: 1 as well as the elements allowing for its expression in the host cell.

A vector, such as a plasmid, can thus be used for transforming host cells. The construction of vectors for transformation of host cells is within the capability of one skilled in the art following standard techniques.

The decision as to whether to use a vector for transforming a cell, or which vector to use, is guided by the method of transformation selected, and by the host cell selected.

Where a naked nucleic acid introduction method is used, then the vector can be the minimal nucleic acid sequences necessary to confer the desired phenotype, without the need for additional sequences.

Possible vectors include the Ti plasmid vectors, shuttle vectors designed merely to maximally yield high numbers of copies, episomal vectors containing minimal sequences necessary for ultimate replication once transformation has occured, transposon vectors, including the possibility of RNA forms of the gene sequences. The selection of vectors and methods to construct them are commonly known to persons of ordinary skill in the art and are described in general technical references (Mullis, K B (1987), Methods in Enzymology).

For other transformation methods requiring a vector, selection of an appropriate vector is relatively simple, as the constraints are minimal. The apparent minimal traits of the vector are that the desired nucleic acid sequence be introduced in a relatively intact state. Thus, any vector which produces a plant carrying the introduced DNA sequence should be sufficient. Also, any vector which introduces a substantially intact RNA which can ultimately be converted into a stably maintained DNA sequence should be acceptable.

For transformation methods within a plant cell, one can cite methods of direct transfer of genes such as direct micro-injection into plant embryos, vacuum infiltration or electroporation, direct precipitation by means of PEG or the bombardment by gun of particules covered with the plasmidic DNA of interest.

It is preferred to transform the plant cell with a bacterial strain, in particular *Agrobacterium,* in particular *Agrobacterium tumefaciens.* In particular, it is possible to use the method described by Ishida et al. (Nature Biotechnology, 14, 745-750, 1996) for the transformation of monocotyledons.

However, any additional attached vector sequences which confer resistance to degradation of the nucleic acid fragment to be introduced, which assists in the process of genomic integration or provides a means to easily select for those cells or plants which are actually, in fact, transformed are advantageous and greatly decrease the difficulty of selecting useable transgenic plants.

The vector can exist, for example, in the form of a phage, a plasmid or a cosmid. The construction of such expression vectors for transformation is well known in the art and uses standard techniques. Mention may be made of the methods described by Sambrook et al. (1989).

For transforming bacteria, a vector is generally defined as being a nucleic acid molecule that possesses elements that allows it to be maintained within said host cell (such as an origin of replication that works in this bacterial host cell).

### SCREENING OF PLANTS RESISTANT OR SUSCEPTIBLE TO CHLOROTOLURON

The identification of the specific gene providing tolerance to chlorotoluron to plants, and of mutations that lead to production of a truncated protein, makes it possible to identify the plants that are tolerant or sensitive to chlorotoluron.

This is of particular interest, as this makes it possible to develop new markers that will be very specific for the herbicide tolerance or susceptibility. These markers will be more reliable than the existing markers, which are co-segregating markers and can be lost *via* a recombination, for instance during meiosis.

The invention thus relates to a method for determining whether a plant is susceptible to chlorotoluron, comprising searching for the presence of a mutation in the gene coding for SEQ ID NO: 1, wherein the mutation leads to formation of an inactive protein or to absence of production of the protein, and wherein a mutation is present on both alleles of the plant. The mutation may not be the same on both alleles, but each mutation should lead to the production of an inactive protein. The inactive protein may be a truncated protein, as the inventors showed that an insertion of a nucleotide in the coding sequence led to a frameshift which would thus produce a truncated inactive protein. However, the mutation may also be the absence of the gene herein described. One can note that the markers used may be associated with the insertion (the insertion is not directly detected, but a variation associated with the insertion is detected: even if this would also be a co-segregating marker, it would be closer to the gene, and thus have fewer chances to have the link between marker and gene broken).

Such method can be applied to a plant population and allows identifying a plant susceptible to chlorotoluron in this population.

In particular, the mutation is an insertion of "T" after position 787 of SEQ ID NO: 3 (SEQ ID NO: 4 represents such insertion with a "T" at position 788).

Similarly, one can use a method for determining whether a plant is resistant to chlorotoluron, comprising determining presence of a functional allele of the gene coding for the protein depicted by SEQ ID NO: 1, wherein presence of a functional allele in the genome of the plant is indicative of the plant resistance or tolerance to chlorotoluron. This can be performed in a plant population to identify the plant that are tolerant to chlorotoluron.

Search for the presence of the mutation or its absence (presence of the functional gene) is performed by any method known in the art, such as sequencing, amplification in particular using adequate primers, or by analysis of the size of amplicons.

In particular, one can perform a sequencing of the locus of SEQ ID NO: 3, an amplification of a region located in SEQ ID NO: 3 or use molecular markers within SEQ ID NO: 3 gene or outside of SEQ ID NO: 3. It is to be noted that molecular markers specific for the resistance/susceptibility to chlorotoluron and closer to the gene responsible for this resistance can be developed, which will be more accurate than the markers currently known. Knowledge of the gene makes it possible to develop new markers, that are more closely genetically linked to the gene.

The methods (transgenesis, restoration of gene function, detection of functional or mutated genes...) are essentially conducted on plant cells and plants. One can perform the methods on monocotyledonous plants or plant cells. It is also possible to perform the methods on dicotyledonous plants or plant cells. Among monocotyledonous plants, one can cite cereals like rice, wheat, barley, sorghum, oat, maize but also sugarcane. Among dicotyledonous plants, one can cite soybean, cotton, tomato, beet, sunflower, or rapeseed.

As indicated above, it is possible, when introducing the gene coding for SEQ ID NO: 1 in a plant or a plant cell, to also introduce other transgenes. Chlorotoluron can then be used as the selection agent, as SEQ ID NO: 1 can be used as a selective marker for cell transformation.

The invention also relates to a transgenic plant cell comprising a gene coding for SEQ ID NO: 1 as a transgene, as well as to a plant comprising a plurality of such plant cells. The invention also relates to a seed from this plant, wherein plant cells of said seed comprise the expression cassette disclosed above and the nucleic acid sequence encoding SEQ ID NO: 1 as a transgene, as well as to a plant grown from such seed. One can also cite a regenerable part, progeny, or asexual propagate of such plant, wherein said regenerable part, progeny, or asexual propagate comprises said expression cassette.

The invention also relates to a method of protecting a crop plant from damage by chlorotoluron, comprising growing a plant transgenic for a nucleic acid encoding SEQ ID NO: 1, and applying chlorotoluron to said plant. In some embodiments, the plant contains another gene imparting resistance to another herbicide, and the method also comprises applying a second herbicide, the first herbicide and said second herbicide being applied sequentially or concurrently.

### MAKING A PLANT SUSCEPTIBLE TO CHLOROTOLURON

The invention also relates to a method for making a plant sensitive to chlorotoluron, comprising inhibiting the expression of the gene coding for the CYP71C4 protein represented by SEQ ID NO: 1 in said plant.

Such method is performed in plants or cells resistant to chlorotoluron. These cells or plants contain a gene coding for SEQ ID NO: 1 (or allelic variants thereof). One can cite, in particular some chlorotoluron tolerant wheat plants, barley plants, or other cereal plants.

It is preferred when such inhibition is performed for both alleles present in the genome of the cells of the plant.

As foreseen in the present invention, a total inhibition of a gene coding for the CYP71C4 protein in a cell indicates either that:
(i) No mRNA is detected in said cell after RNA isolation and reverse transcription or Northern Blot..
(ii) No functional protein is produced in said cell. No functional protein is produced in absence of the mRNA (see (i)). In other cases, mRNA may be present but leads to the production of a truncated or non-functional protein (as an illustration when the T is inserted within SEQ ID NO: 3 and introduces a frameshift), or when the mRNA is incomplete, in particular in case of the presence of a mutation within the gene, in an intron or in an exon). Truncated proteins can be detected by isolation of the proteins, Western Blot and detection of the size of the protein with an antibody (polyclonal or monoclonal) directed against the CYP71C4 protein.

A partial inhibition of a gene coding for CYP71C4 in a cell indicates that mRNA can be detected in said cell after RNA isolation and reverse transcription or Northern Blot, but at a lower level than that detected in a cell which do not bear the element introduced in the cell for inhibiting CYP71C4 expression. In particular, partial inhibition is obtained when a lower level of mRNA is detected after RNA isolation and reverse transcription.

In one embodiment, expression and/or activity of CYP71C4 is inhibited by mutagenesis of the gene coding for said protein.

The mutagenesis of the gene can take place at the level of the coding sequence or of the regulatory sequences for expression, in particular of the promoter. It is, for example, possible to delete all or part of said gene and/or to insert an exogenous sequence.

By way of example, mention will be made of insertional mutagenesis: a large number of individuals derived from a plant that is active in terms of the transposition of a transposable element (such as the AC or Mutator elements in maize) are produced, and the plants in which there has been an insertion in the gene coding for CYP71C4 are selected, for example by PCR.

It is also possible to introduce one or more point mutations with physical (for example radiations) or chemical agents, such as seed treatment with EMS or sodium azide, site-directed DNA nucleases or gamma irradiation. The consequences of these mutations may be to shift the reading frame and/or to introduce a stop codon into the sequence and/or to modify the level of transcription and/or of translation of the gene. In this context, use may in particular be made of techniques of the "TILLING" type (Targeting Induced Local Lesions IN Genomes; McCALLUM et al., Plant Physiol., 123, 439-442, 2000). Such mutated plants are then screened, in particular by PCR, using primers located in the target gene. One can also use other screening methods, such as Southern Blots or the AIMS method that is described in WO 99/27085 (this method makes it possible to screen for insertion), by using probes that are specific of the target genes, or through methods detecting point mutations or small insertions / deletions by the use of specific endonucleases (such as Cel I, Endo I, which are described in WO 2006/010646).

One can also use the CRISPR-Cas system (in particular the base editing) to introduce a mutation, an insertion or a deletion in the sequence of the gene coding for CYP71.

One can also use the RNA interference (RNAi) method, which is particularly efficient for extinction of genes in plants (Helliwell and Waterhouse, 2003). This method is well known by the person skilled in the art, and comprises transformation of the plant with a construct producing, after transcription, a double-stranded duplex RNA, one of the strand of which being complementary of the mRNA of the target gene.

In another embodiment, inhibition of the CYP71C4 expression is due to the presence in cells of said plant of an antisense, or overexpression (leading to co-suppression), or RNAi construct. The DNA constructs used in these methods are introduced in the genome of said plant through methods known in the art.

In particular, inhibition may be obtained by transforming the plant with a vector containing a sense or antisense construct. These two methods (co-suppression and antisense method) are well known in the art to permit inhibition of the target gene. It is to be noted that the data obtained in Arabidopsis demonstrates that the co-suppression approach seems adapted for this gene.

### USING CHLOROTOLURON SUSCEPTIBLE PLANTS FOR MAKING HYBRID WHEAT

The invention relates also to a method for facilitating the production of hybrid wheat seeds as described in the patent application EP 1934 354 B1 in which the herbicide tolerance gene is the Cyp71c4 gene and the herbicide to kill male or female reproductive tissue is Chlorotoluron.

In a such strategy, a miRNA recognition site or 2 or 3 mi RNA recognition sites is/are associated with the messenger RNA encoding the CYP71C4 protein imparting tolerance to an herbicide by gene editing and/or homologous recombination. The miRNA recognition sites is /are recognizable by a natural mature miRNA that are endogenous to and specifically expressed in male reproductive key tissues like tapetum, microspore mother cells. Those are well known in the literature including in cereals especially wheat. This will be sufficient to induce male sterility in the plant treated by Chlorotoluron as it is known as a systemic herbicide. Such a plant can be multiply without any herbicide addition and rendered male sterile and so female in hybrid production step. The male plants of the hybrid production scheme contain the natural SU1 resistant allele at homozygous state ensuring pollen production and giving hybrid seeds that will be totally resistant to chlorotoluron. Reciprocally the addition of mi RNA target sites for miRNAs expressed in female tissue could lead to an application where we render plants to be female sterile.

Combination of the two types of SU1 modifications can allow hybrid production schema where you are getting only hybrid seeds harvested from the hybrid production field.

### ESSENTIAL SEQUENCES OF THE APPLICATION

**Table 1. Sequences of the application.**

| **SEQ ID NO** | **Sequence** |
|---|---|
| 1 functional protein wheat | MAAQTVLPLLLSPQALLLYLLLIPLVILLRATSTRPAGERAAADNHG GRPLPSPPNKLPLIGHLHLVGPDPHVSLAILAAKHGDGGLMLLRLG QTRALVVSSPRAAEAVLRTQDHVFASRPPNALVDALLDGGADVAF APYGEFWRQARRLVTTHLLSARKLHSLRRGREGEARLVMAKIQGA AAAGAPVDMTRLLGSFTNDIACRAVSGKFFREEGRNELFREAIDG NVAAFGGFSLLDYFPGLAKVGVLTRVVFAKNSQLKRKWDELLDKII DDHVSKASAQEQHHKHDQQEDQETDFVDVLLSLQEEYSLTRQHIK AILMDMFVAGTDTSYIVLECAMAELMRKPNLMAKLQAEVREKTPK GQQMVTEDDLASMAYLKATVKETLRLHPPLPLLLPHLSMTECDIDG YKVPARTRVIVNAWALGRDPGSWEAADKFMPERFVDVVSPDFKG RDFQFLPFGAGRRICPGINFGMASVEIMLANLVYCYDWELPDGMR QEDLDMTDVFGMTMRRKEKLFLVPRTRDVNHA |
| 2 cDNA wheat (functional) | ATGGCGGCTCAAACAGTGCTACCACTGCTCTTATCTCCCCAAGC ACTATTGCTCTATCTCTTGCTCATCCCTCTGGTGATCCTCCTCC GGGCAACAAGCACGAGACCGGCCGGCGAGCGAGCCGCAGCG GACAACCATGGGGGCCGTCCCCTCCCTTCGCCTCCGAACAAGC TCCCTCTCATCGGCCACCTGCACCTGGTCGGCCCCGACCCGCA CGTCTCCCTCGCCATCCTCGCCGCCAAGCACGGCGACGGCGG CCTCATGCTCCTCCGCCTCGGCCAGACGCGCGCCCTCGTCGTC |
| | TCCTCCCCGCGCGCCGCCGAGGCGGTCCTGCGCACGCAGGAC CACGTCTTCGCCTCGCGGCCTCCGAACGCGCTCGTCGACGCC CTCCTGGACGGCGGCGCCGACGTCGCCTTCGCCCCCTACGGC GAGTTCTGGCGGCAGGCCAGGAGGCTGGTCACCACGCACCTG CTCAGCGCCAGGAAGCTGCACTCGCTCCGCCGCGGCCGCGAG GGGGAGGCGCGCCTGGTGATGGCCAAGATCCAGGGCGCGGC GGCCGCCGGCGCCCCCGTGGACATGACGAGGCTGCTCGGCAG CTTCACCAACGACATAGCGTGCCGCGCCGTGTCCGGGAAGTTC TTCCGGGAGGAAGGCCGGAACGAGCTGTTCAGGGAGGCGATC GACGGGAACGTGGCCGCGTTTGGCGGGTTCAGCCTGCTGGAC TACTTCCCGGGCCTGGCCAAGGTGGGCGTGCTCACGCGAGTG GTTTTCGCCAAGAACAGTCAGTTGAAGAGGAAATGGGACGAGC TGCTCGACAAGATCATCGACGACCATGTCAGCAAAGCATCGGC ACAGGAGCAACACCACAAGCACGATCAGCAAGAAGATCAAGAG ACAGACTTCGTCGATGTGCTCCTCAGTCTTCAGGAGGAGTACA GTCTCACCAGACAGCATATCAAGGCCATACTGATGGACATGTTT GTGGCAGGCACAGATACGTCATACATAGTTCTTGAGTGCGCCA TGGCTGAGCTCATGCGAAAGCCGAACCTCATGGCCAAACTTCA AGCTGAGGTGAGGGAAAAGACACCCAAGGGCCAACAAATGGT GACCGAGGACGACTTGGCCAGCATGGCCTACCTAAAGGCCACC GTGAAGGAAACGCTCCGGCTACACCCACCGCTGCCGCTCCTCC TCCCGCACCTCTCCATGACCGAATGCGACATCGACGGCTATAA AGTCCCCGCCAGAACCCGTGTCATCGTAAACGCTTGGGCTCTT GGTAGAGACCCTGGATCGTGGGAGGCTGCCGACAAGTTCATGC CGGAGAGGTTTGTGGACGTCGTGTCCCCCGACTTCAAAGGGAG GGATTTTCAGTTTCTGCCGTTTGGAGCTGGCCGAAGGATATGTC CCGGGATAAATTTTGGGATGGCGTCTGTGGAGATCATGCTTGC CAACCTTGTCTATTGTTATGATTGGGAGCTCCCGGACGGCATGC GGCAAGAGGACCTGGATATGACTGATGTGTTTGGCATGACGAT GCGTCGGAAGGAGAAGTTATTTCTTGTCCCGAGAACACGAGAT GTTAATCATGCATAA |
| 3 genomic sequence wheat | ctatatcacttggcttagacttggttaagtctcagttgactgagatctagctacatccttatattaaa gatccaacggctgctctactttctccttccacgaaattaacttacccaaattgtaaatttagtcaac ttacatgtaactattgcagaaggaaggaaaaacatatttcccatgcattggcaggtggataag |
| (functional) Exon 1: 445-1413 Exon 2: 1508-2134 | atacgtacatatgtgatcatggttgcgccgcgcatgtccatcgatggagaaattcaagtccacc accactaaacgatgaacagaagtggagaaggacacgcaccaaactccaaagtccaaacc tcattgtcacgtagcaaatcgcgagcatgggctacgcatccagtcgtggtcagaagatcacag tgatcactcttgtcgaccagctacaaatacgacaccaagggagggtgctacaagcaggccA TGGCGGCTCAAACAGTGCTACCACTGCTCTTATCTCCCCAAGCA CTATTGCTCTATCTCTTGCTCATCCCTCTGGTGATCCTCCTCCG GGCAACAAGCACGAGACCGGCCGGCGAGCGAGCCGCAGCGG ACAACCATGGGGGCCGTCCCCTCCCTTCGCCTCCGAACAAGCT CCCTCTCATCGGCCACCTGCACCTGGTCGGCCCCGACCCGCA CGTCTCCCTCGCCATCCTCGCCGCCAAGCACGGCGACGGCGG CCTCATGCTCCTCCGCCTCGGCCAGACGCGCGCCCTCGTCGTC TCCTCCCCGCGCGCCGCCGAGGCGGTCCTGCGCACGCAGGAC CACGTCTTCGCCTCGCGGCCTCCGAACGCGCTCGTCGACGCC CTCCTGGACGGCGGCGCCGACGTCGCCTTCGCCCCCTACGGC GAGTTCTGGCGGCAGGCCAGGAGGCTGGTCACCACGCACCTG CTCAGCGCCAGGAAGCTGCACTCGCTCCGCCGCGGCCGCGAG GGGGAGGCGCGCCTGGTGATGGCCAAGATCCAGGGCGCGGC GGCCGCCGGCGCCCCCGTGGACATGACGAGGCTGCTCGGCAG CTTCACCAACGACATAGCGTGCCGCGCCGTGTCCGGGAAGTTC TTCCGGGAGGAAGGCCGGAACGAGCTGTTCAGGGAGGCGATC GACGGGAACGTGGCCGCGTTTGGCGGGTTCAGCCTGCTGGAC TACTTCCCGGGCCTGGCCAAGGTGGGCGTGCTCACGCGAGTG GTTTTCGCCAAGAACAGTCAGTTGAAGAGGAAATGGGACGAGC TGCTCGACAAGATCATCGACGACCATGTCAGCAAAGCATCGGC ACAGGAGCAACACCACAAGCACGATCAGCAAGAAGATCAAGAG ACAGACTTCGTCGATGTGCTCCTCAGTCTTCAGGAGGAGTACA GTCTCACCAGACAGCATATCAAGGCCATACTGATGgtaaatctcgtctc atacgtagctagcttattatggtaattaaatgtggtcatattttaagtgtgctgacgcttcagtttgaa cccatacacagGACATGTTTGTGGCAGGCACAGATACGTCATACATA GTTCTTGAGTGCGCCATGGCTGAGCTCATGCGAAAGCCGAACC TCATGGCCAAACTTCAAGCTGAGGTGAGGGAAAAGACACCCAA GGGCCAACAAATGGTGACCGAGGACGACTTGGCCAGCATGGC CTACCTAAAGGCCACCGTGAAGGAAACGCTCCGGCTACACCCA CCGCTGCCGCTCCTCCTCCCGCACCTCTCCATGACCGAATGCG |
| | ACATCGACGGCTATAAAGTCCCCGCCAGAACCCGTGTCATCGT AAACGCTTGGGCTCTTGGTAGAGACCCTGGATCGTGGGAGGCT GCCGACAAGTTCATGCCGGAGAGGTTTGTGGACGTCGTGTCCC CCGACTTCAAAGGGAGGGATTTTCAGTTTCTGCCGTTTGGAGCT GGCCGAAGGATATGTCCCGGGATAAATTTTGGGATGGCGTCTG TGGAGATCATGCTTGCCAACCTTGTCTATTGTTATGATTGGGAG CTCCCGGACGGCATGCGGCAAGAGGACCTGGATATGACTGATG TGTTTGGCATGACGATGCGTCGGAAGGAGAAGTTATTTCTTGTC CCGAGAACACGAGATGTTAATCATGCATAAtatctttcatgaattatgtgcac ttgtaaagctagatattacactagtatatatggttttcccagttttcaagagggtatgtgggaaaca ctccaggtactcacattctgccacacaaaaactaccaaccgtcatcccatttctagtccacggc gatcaaatgggttttattgatggtagaaatatcgtggagcacgttatgtttgttgcctacctcatca agtcttgccatggtaggaaggagcaacccatggtcattaagcttgactttcaaaaaacattcga ctcagtttcttggacctcacttatatccactctaagactttgtggtttccaaccattttttggtagaaac catcctttctacaggcagaacgatatgagacaaggcaagcccctttcaccttatctcttcattatt gttgttgatattcttcaacaactaataaataaaaattatagtcacaattctctctcctatccatttaca actacctaccaccaacgacaatagaatacatgggacgacgcccttatcatagttaagttctca ccctaagaccttgaaaccctaaaacacatcttagacgacttcgcttgaaccctgaattgatctg acgtgcttggcatcagatctcatcatcgcaaacagatgatgagaaaccctaacctcagcgcc caaggagacgacaggaatttacgttggcactctgttggttccgtccagaaggacacactcga ggaggatcggagctcagaagacctactcaaagaagaggtgctgtcatccgcttgcacaccgt cctgcgaggactaaaaaccctaacctaaactactagtcggagtcgaggcatcgaaattctcc |
| 4 genomic sequence wheat (defective) | ctatatcacttggcttagacttggttaagtctcagttgactgagatctagctacatccttatattaaa gatccaacggctgctctactttctccttccacgaaattaacttacccaaattgtaaatttagtcaac ttacatgtaactattgcagaaggaaggaaaaacatatttcccatgcattggcaggtggataag atacgtacatatgtgatcatggttgcgccgcgcatgtccatcgatggagaaattcaagtccacc accactaaacgatgaacagaagtggagaaggacacgcaccaaactccaaagtccaaacc tcattgtcacgtagcaaatcgcgagcatgggctacgcatccagtcgtggtcagaagatcacag tgatcactcttgtcgaccagctacaaatacgacaccaagggagggtgctacaagcaggccA TGGCGGCTCAAACAGTGCTACCACTGCTCTTATCTCCCCAAGCA CTATTGCTCTATCTCTTGCTCATCCCTCTGGTGATCCTCCTCCG GGCAACAAGCACGAGACCGGCCGGCGAGCGAGCCGCAGCGG ACAACCATGGGGGCCGTCCCCTCCCTTCGCCTCCGAACAAGCT CCCTCTCATCGGCCACCTGCACCTGGTCGGCCCCGACCCGCA |
| | CGTCTCCCTCGCCATCCTCGCCGCCAAGCACGGCGACGGCGG CCTCATGCTCCTCCGCCTCGGCCAGACGCGCGCCCTCGTCGTC TCCTCCCCGCGCGCCGCCGAGGCGGTCCTGCGCACGCAGGAC CTACGTCTTCGCCTCGCGGCCTCCGAACGCGCTCGTCGACGCC CTCCTGGACGGCGGCGCCGACGTCGCCTTCGCCCCCTACGGC GAGTTCTGGCGGCAGGCCAGGAGGCTGGTCACCACGCACCTG CTCAGCGCCAGGAAGCTGCACTCGCTCCGCCGCGGCCGCGAG GGGGAGGCGCGCCTGGTGATGGCCAAGATCCAGGGCGCGGC GGCCGCCGGCGCCCCCGTGGACATGACGAGGCTGCTCGGCAG CTTCACCAACGACATAGCGTGCCGCGCCGTGTCCGGGAAGTTC TTCCGGGAGGAAGGCCGGAACGAGCTGTTCAGGGAGGCGATC GACGGGAACGTGGCCGCGTTTGGCGGGTTCAGCCTGCTGGAC TACTTCCCGGGCCTGGCCAAGGTGGGCGTGCTCACGCGAGTG GTTTTCGCCAAGAACAGTCAGTTGAAGAGGAAATGGGACGAGC TGCTCGACAAGATCATCGACGACCATGTCAGCAAAGCATCGGC ACAGGAGCAACACCACAAGCACGATCAGCAAGAAGATCAAGAG ACAGACTTCGTCGATGTGCTCCTCAGTCTTCAGGAGGAGTACA GTCTCACCAGACAGCATATCAAGGCCATACTGATGgtaaatctcgtctc atacgtagctagcttattatggtaattaaatgtggtcatattttaagtgtgctgacgcttcagtttgaa cccatacacagGACATGTTTGTGGCAGGCACAGATACGTCATACATA GTTCTTGAGTGCGCCATGGCTGAGCTCATGCGAAAGCCGAACC TCATGGCCAAACTTCAAGCTGAGGTGAGGGAAAAGACACCCAA GGGCCAACAAATGGTGACCGAGGACGACTTGGCCAGCATGGC CTACCTAAAGGCCACCGTGAAGGAAACGCTCCGGCTACACCCA CCGCTGCCGCTCCTCCTCCCGCACCTCTCCATGACCGAATGCG ACATCGACGGCTATAAAGTCCCCGCCAGAACCCGTGTCATCGT AAACGCTTGGGCTCTTGGTAGAGACCCTGGATCGTGGGAGGCT GCCGACAAGTTCATGCCGGAGAGGTTTGTGGACGTCGTGTCCC CCGACTTCAAAGGGAGGGATTTTCAGTTTCTGCCGTTTGGAGCT GGCCGAAGGATATGTCCCGGGATAAATTTTGGGATGGCGTCTG TGGAGATCATGCTTGCCAACCTTGTCTATTGTTATGATTGGGAG CTCCCGGACGGCATGCGGCAAGAGGACCTGGATATGACTGATG TGTTTGGCATGACGATGCGTCGGAAGGATAAGTTATTTCTTGTC CCGAGAACACGAGATGTTAATCATGCATAATatctttcatgaattatgtgca |
| | cttgtaaagctagatattacactagtatatatggttttcccagttttcaagagggtatgtgggaaac actccaggtactcacattctgccacacaaaaactaccaaccttcatcccatttctagtccacga cgatcaaatgggttttattgatggtagaaatatcgtggagcacgttatgtttgttgcctacctcatca agtcttgccatggtaggaaggagcaacccatggtcattaagcttgactttcaaaaaacattcga ctcagtttcttggacctcacttatatccactctaagactttgtggtttccaaccattttttggtagaaac catcctttctaccggcagaacgatatgagacaaggcaagcccctttcaccttatctcttcattatt gttgttgatattcttcaacaactaataaataaaaattatagtcacaattctctctcctatccatttaca actacctaccaccaacgacaatagaatacatgggacgacgcccttatcatagttaagttctca ccctaagaccttgaaaccctaaaacacatcttagacgacttcgcttgaaccctgaattgatctg acgtgcttggcatcagatctcaccatcgcaaacagatgatgagaaaccctaacctcagcgcc caaggagacgacaggaatttacgttggcactctgttggttccgtccagacggacacactcgag gaggatcggagctcagaagacctactcaaagaagaggtgctgtcatccgcctgcacaccgt cctgcgaggactaaaaaccctaacctaaactactagtcggggtcgacacaccgaaattctcc |
| 5 defective protein wheat | MAAQTVLPLLLSPQALLLYLLLIPLVILLRATSTRPAGERAAADNHG GRPLPSPPNKLPLIGHLHLVGPDPHVSLAILAAKHGDGGLMLLRLG QTRALVVSSPRAAEAVLRTQDLRLRLAASERARRRPPGRRRRRRL RPLRRVLAAGQEAGHHAPAQRQEAALAPPRPRGGGAPGDGQDP GRGGRRRPRGHDEAARQLHQRHSVPRRVREVLPGGRPERAVQG GDRRERGRVWRVQPAGLLPGPGQGGRAHASGFRQEQSVEEEM GRAARQDHRRPCQQSIGTGATPQARSARRSRDRLRRCAPQSSG GVQSHQTAYQGHTDGHVCGRHRYVIHSS |
| 6 functional protein (barley) | MAAQPVLPLLLSTQALLLYLLLIPLLILLRATSRRSAGERAAVDNHG GRPLPSPPNKLPLIGHLHMVGSDPHVSLAALAAKHGDGGLMLLRL GQTRALVVSSPRAAEAVLRTQDHVFASRPTNAVVDALLDGGADIA FVPYGEFWRQARRLVTTHLLSARKVHSLRRGREEEARLVMAKIQG AAAAGATVDMTRLLGAFTNDIVCRAVSGKFFREEGRNELFREVIDG NVAAFGGFNLLDYYPGLAKVSMLRRLVFAKTNRLKRKWDDLLDKII VDHVSQKASPREQHRDRDQQEDEEETDFVDVLLGLQEEYNLTTE HIKAILMDMFVAGTDTSYIVLECAMAELMRKPNLMTKLQAEVGEKT PKGQQMVTEDDLGSMAYLKAVVKETLRLHTPVPLLLPHLSMAECN IDGYTVPAETRVVVNAWALGRDHGSWEAAGEFMPERFGDIVSPD FKGRDFQFLPFGAGRRMCPGINFAMATVEIMLANLVYCYDWELPG GMRQEDLDMTDVFGMTMRRKEKLFLVPISRNVNRA |
| 7 cDNA | ATGGCGGCTCAACCTGTGCTGCCACTGCTCTTATCTACCCAAG |
| barley (functional) | CACTGTTGCTCTATCTCTTGCTCATCCCTCTGCTGATCCTCCTC CGGGCAACGAGCAGGAGATCGGCCGGCGAGCGAGCTGCAGTG GACAACCATGGGGGCCGTCCCCTCCCTTCGCCTCCCAACAAGC TCCCTCTCATCGGCCACCTGCACATGGTCGGCTCCGACCCCCA CGTCTCCCTCGCCGCCCTCGCCGCTAAGCACGGCGACGGCGG TCTCATGCTCCTCCGCCTCGGCCAGACGCGCGCCCTCGTCGTC TCCTCCCCGCGCGCCGCCGAGGCGGTCCTGCGCACGCAGGAC CACGTCTTCGCCTCGCGGCCGACGAACGCGGTCGTCGACGCC CTCCTCGACGGCGGCGCTGACATCGCCTTCGTCCCCTACGGC GAGTTCTGGCGGCAGGCGAGGAGGCTGGTCACCACGCACCTG CTCAGCGCCAGGAAGGTGCACTCCCTCCGCCGCGGCCGCGAG GAGGAAGCGCGCCTGGTGATGGCCAAGATCCAGGGCGCGGCG GCCGCCGGCGCCACCGTGGACATGACGAGGCTGCTCGGCGCC TTCACCAACGACATAGTGTGCCGCGCCGTGTCCGGGAAGTTCT TCCGGGAGGAAGGCCGGAACGAGCTGTTCAGGGAGGTCATCG ACGGGAACGTGGCCGCGTTTGGCGGGTTCAACCTGCTGGACTA CTACCCGGGCCTGGCCAAGGTGAGCATGCTCAGGCGACTGGT GTTTGCCAAGACCAACCGGTTGAAGAGGAAATGGGACGACCTG CTCGATAAGATCATCGTCGATCATGTCAGCCAAAAAGCATCGCC ACGGGAGCAGCACCGCGACCGCGATCAGCAAGAAGATGAAGA AGAGACAGACTTCGTCGATGTGCTCCTCGGTCTTCAGGAGGAG TACAATCTCACCACAGAGCATATAAAGGCCATATTGATGGACAT GTTTGTGGCAGGCACAGATACTTCATACATAGTTCTCGAGTGCG CCATGGCTGAGCTCATGCGAAAGCCGAACCTCATGACCAAACT TCAAGCTGAGGTGGGGGAGAAGACACCCAAGGGCCAACAAAT GGTGACCGAGGACGACTTGGGCAGCATGGCCTACCTGAAGGC CGTCGTGAAGGAGACGCTCCGGCTACACACACCGGTGCCGCT CCTCCTCCCGCACCTCTCCATGGCCGAATGCAACATCGACGGT TATACAGTTCCCGCTGAAACCCGTGTCGTCGTAAACGCTTGGG CTCTTGGTAGAGACCATGGATCGTGGGAGGCTGCCGGCGAGTT CATGCCGGAGAGGTTTGGCGACATCGTGTCCCCCGACTTCAAA GGGAGGGATTTCCAGTTTCTTCCGTTTGGAGCTGGACGAAGGA TGTGCCCCGGGATAAATTTCGCGATGGCTACTGTGGAGATCAT GCTCGCTAACCTTGTCTATTGTTATGATTGGGAGCTTCCGGGTG |
| | GAATGCGGCAAGAGGACCTCGATATGACAGATGTGTTTGGCAT GACGATGCGCCGGAAGGAGAAGTTGTTTCTTGTCCCGATATCG AGGAATGTTAATCGTGCATAA |
| 8 functional genomic (barley) Exon 1: 915 to 1889 Exon 1: 1984 to 2610 | CTTCGGGAATTTTGTTCTACTTCAAATAATTTGGTGCTTCACAAA AATTTCGAAAATTTAAGAAAGTGGGAATTTTGAAATAGAATGTTT AATAAATTATAAAATGTTTGTGGATTCAAAAATACCCGCGATTTT GCAGAAAATGTTAGAAATTTTGAAAACAAATATTTGAGAAATCAA ACAATGTTCATGATTTTTTAAAAGTTCATGAAGTACTTTTTATAAT GAAATTGTGCAAAATTTTGTCAATTCTAAAAATGATCATGAATGG AAAAATATTCTAAAAGTTCAAAAAAACAATGATTTGTGACTTATA AAATAGTTTTTCATTTATAAAATGTTTGCTGATTTAGAAAATTATC ATGCATTTTTTTGTCCCGTTGCAACGCACGGGCCCTTTTGTTAG TAAGTATACAAAAGTTTTAGTTGGTTTTAAATTTTGTGAAAACAAA ATGTGGCAAGGTGGCACAAACACAGGGTGTGACATTTTCCTTAA AAAAAACCTTTCTTCTTGGTATAATCTTCTTTCTTCTCCCATCGTT TCTCCTTCCACAAAATCGAGCTATCCAGATTGTAAATTTAGTCAA CTTACGTATGGCTATAGCGCAAAAAAAAAAAACATATTTTCCATG CATTTGCAGGTGGATAAGATACGTACGTATGTGATCATGGTTCC GCCGCGCCTATCCATCGATCGACAAATTCAAGTCCCACTACGTA CTAAACGTTGAACAGAAGAGGAGAAGGACACACTCCAAACTCC AAAGACTCCAAACCTCATTGTGAGGTAGCAAATCGGGCCATGT GCTACACATCCATCCGTGGTCCGTACTCGATCACTCTTCTAGTA CTCGACCAGCTACAAATACTGACACCAAGGGAGGGTGCATGTG CCGTACTGCTAGCAAGCAGGCATGGCGGCTCAACCTGTGCTGC CACTGCTCTTATCTACCCAAGCACTGTTGCTCTATCTCTTGCTCA TCCCTCTGCTGATCCTCCTCCGGGCAACGAGCAGGAGATCGGC CGGCGAGCGAGCTGCAGTGGACAACCATGGGGGCCGTCCCCT CCCTTCGCCTCCCAACAAGCTCCCTCTCATCGGCCACCTGCAC ATGGTCGGCTCCGACCCCCACGTCTCCCTCGCCGCCCTCGCC GCTAAGCACGGCGACGGCGGTCTCATGCTCCTCCGCCTCGGC CAGACGCGCGCCCTCGTCGTCTCCTCCCCGCGCGCCGCCGAG GCGGTCCTGCGCACGCAGGACCACGTCTTCGCCTCGCGGCCG ACGAACGCGGTCGTCGACGCCCTCCTCGACGGCGGCGCTGAC ATCGCCTTCGTCCCCTACGGCGAGTTCTGGCGGCAGGCGAGG |
| | AGGCTGGTCACCACGCACCTGCTCAGCGCCAGGAAGGTGCAC TCCCTCCGCCGCGGCCGCGAGGAGGAAGCGCGCCTGGTGATG GCCAAGATCCAGGGCGCGGCGGCCGCCGGCGCCACCGTGGA CATGACGAGGCTGCTCGGCGCCTTCACCAACGACATAGTGTGC CGCGCCGTGTCCGGGAAGTTCTTCCGGGAGGAAGGCCGGAAC GAGCTGTTCAGGGAGGTCATCGACGGGAACGTGGCCGCGTTT GGCGGGTTCAACCTGCTGGACTACTACCCGGGCCTGGCCAAG GTGAGCATGCTCAGGCGACTGGTGTTTGCCAAGACCAACCGGT TGAAGAGGAAATGGGACGACCTGCTCGATAAGATCATCGTCGA TCATGTCAGCCAAAAAGCATCGCCACGGGAGCAGCACCGCGAC CGCGATCAGCAAGAAGATGAAGAAGAGACAGACTTCGTCGATG TGCTCCTCGGTCTTCAGGAGGAGTACAATCTCACCACAGAGCA TATAAAGGCCATATTGATGGTAATTAAATCCTCTCATGCCGTCAT ACTCCTACGTGCGTAGCTGGCTGATTTAAGTGTGCTGACGCTTC AGGTTGAACTGAATCCGTACGCAGGACATGTTTGTGGCAGGCA CAGATACTTCATACATAGTTCTCGAGTGCGCCATGGCTGAGCTC ATGCGAAAGCCGAACCTCATGACCAAACTTCAAGCTGAGGTGG GGGAGAAGACACCCAAGGGCCAACAAATGGTGACCGAGGACG ACTTGGGCAGCATGGCCTACCTGAAGGCCGTCGTGAAGGAGAC GCTCCGGCTACACACACCGGTGCCGCTCCTCCTCCCGCACCTC TCCATGGCCGAATGCAACATCGACGGTTATACAGTTCCCGCTG AAACCCGTGTCGTCGTAAACGCTTGGGCTCTTGGTAGAGACCA TGGATCGTGGGAGGCTGCCGGCGAGTTCATGCCGGAGAGGTT TGGCGACATCGTGTCCCCCGACTTCAAAGGGAGGGATTTCCAG TTTCTTCCGTTTGGAGCTGGACGAAGGATGTGCCCCGGGATAA ATTTCGCGATGGCTACTGTGGAGATCATGCTCGCTAACCTTGTC TATTGTTATGATTGGGAGCTTCCGGGTGGAATGCGGCAAGAGG ACCTCGATATGACAGATGTGTTTGGCATGACGATGCGCCGGAA GGAGAAGTTGTTTCTTGTCCCGATATCGAGGAATGTTAATCGTG CATAATATTTGGCACAAATTTTGTGCATGGTAAGGCTAGATGGT GTATTTGTATGTATGGTTTTCCAGCTTTATCGAAAAATACTTTCG CCCCGTTTTATAGATGAAGCAAACCACCGAACCACAACATCCAA CAACAGTCACAATAACACATACACACGCCGCACTAGAGGTAGC ACAACAACACAAGGTACGAAGGTAACGTTGAGGGCACAACACA |
| | ACAAGCCCAAACAAAGGAAAACAACAAGGCGGCGCTGCAAAAC AAAAATGATCCTCTAATCCGGCTTCGGAGGTGGAGGAGGGAGC GGCCGCGCCAAGCGGAGAGCCATTGCAAGAAG |

| | |
|---|---|
| With regards to SEQ ID NO: 3, one can note Nucleotides 1-444: 5' UTR, promoter Nucleotides 445-1413: first exon Nucleotides 1414-1507: intron Nucleotides 1508-2134: second exon Nucleotides 2135-2999: 3' UTR | |

### DESCRIPTION OF THE FIGURES

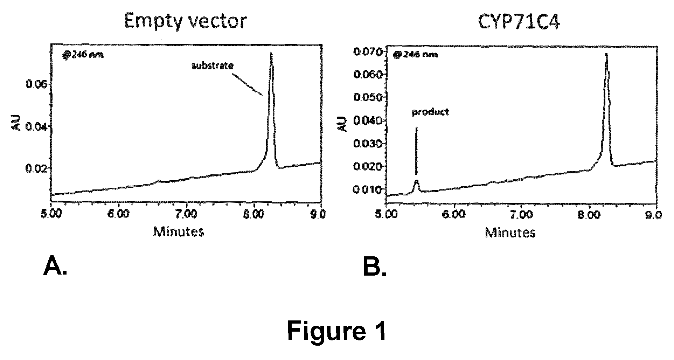

Figure 1: HPLC analysis of the products produce by yeast transformed with an empty vector (A) and with a vector allowing expression of SEQ ID NO: 1 (B). the figure shows the chromatograms on HPLC-UV of microsome extract after incubation with chlorotoluron. HPLC AU: absorbance at 246 nm (optimal wavelength for product detection). The pic labelled "substrate" is the main product obtained using chlorotoluron as substrate. Pic "product" corresponds to a hydroxylated version of the substrate.

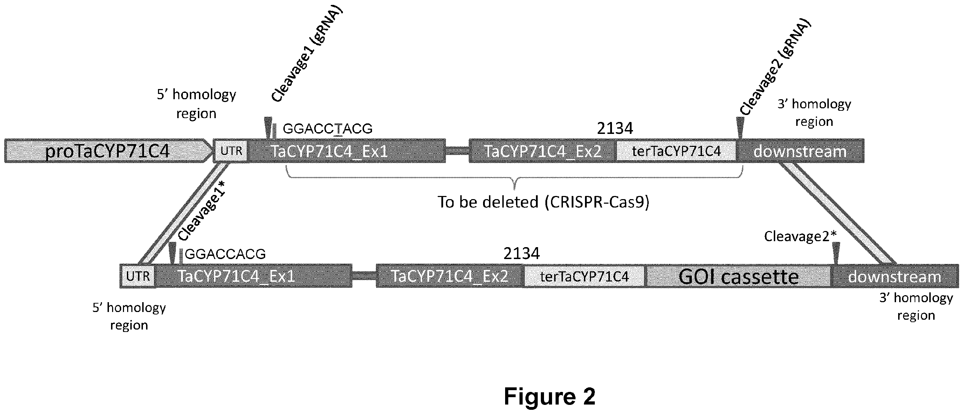

Figure 2: schematic representation of use of the CYP71C4 locus as a landing pad for introducing a transgene from a donor sequence, (bottom) within the genome (up) downstream of the gene while correcting a defective mutation in the gene to make it functional (deletion of the T in the GGACCTACG sequence). proTaCYP71C4 / UTR: promoter /5' UTR of the gene coding for SEQ ID NO: 1 (includes 1-444 of SEQ ID NO: 3). TaCYP71C4_Ex1 and TaCYP71C4_Ex2: exons 1 and 2 of the gene coding for SEQ ID NO: 1 (Nucleotides 445-1413 and 1508-2134 of SEQ ID NO: 3). terTaCYP71C4: terminator sequence of the gene coding for SEQ ID NO: 1 (includes nt 2135-2999 of SEQ ID NO: 3). GOI cassette indicates the Gene Of Interest (transgene). Triangles represent sequences targeted by gRNA for cleavage of the genomic sequence. The link between the homologous sequences between the donor and genomic sequences is schematized.

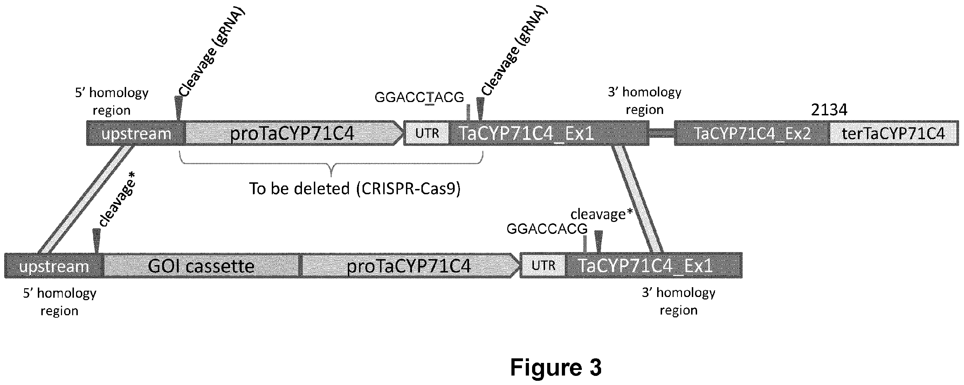

Figure 3: schematic representation of use of the CYP71C4 locus as a landing pad for introducing a transgene from a donor sequence, (bottom) within the genome (up) upstream of the gene while correcting a defective mutation in the gene to make it functional (deletion of the T in the GGACCTACG sequence). proTaCYP71C4 / UTR: promoter /5' UTR of the gene coding for SEQ ID NO: 1 (includes 1-444 of SEQ ID NO: 3). TaCYP71C4_Ex1 and TaCYP71C4_Ex2: exons 1 and 2 of the gene coding for SEQ ID NO: 1 (Nucleotides 445-1413 and 1508-2134 of SEQ ID NO: 3). terTaCYP71C4: terminator sequence of the gene coding for SEQ ID NO: 1 (includes nt 2135-2999 of SEQ ID NO: 3). GOI cassette indicates the Gene Of Interest (transgene). Triangles represent sequences targeted by gRNA for cleavage of the genomic sequence. The link between the homologous sequences between the donor and genomic sequences is schematized.

### EXAMPLES

### Example 1. Fine mapping of Su1 locus through GWAS/QTL analysis and Identification of Su1 causal gene

The chlorotoluron resistant gene '*Su1*' in hexaploid bread wheat *(Triticum aestivum* L.) was first mapped close to the chromosome 6B centromere by Krugman *et al. (op. cit*.) using RFLP markers within a biparental population Chinese Spring' x 'CS CAP6B' of 58 F4 SSD lines. The authors concluded that the Su1 locus where within the marker interval defined by Nor2 (chrom 6B short arm) and Xpsr371 (chrom 6B long arm).

Using specific and proprietary data, including various sequences of wheat lines tolerant or susceptible to chlorotoluron (206 lines (67% tolerant and 33% susceptible)), the inventors were able to fine-map the interval and identified a P450 candidate gene within the interval by running a GWAS analysis.

The inventors were first able to reduce the locus to a region of 4.5 cM (centimorgan) on chromosome 6BS. Using internal sequence data (a synthetic wheat genome based on synteny relationship between cereals), the inventors determined that the interval could contain 600 to 700 genes. However, the information was not sufficient to identify a candidate.

It was thus necessary to perform a further analysis on a larger panel consisting of 379 lines (67% tolerant and 33% susceptible) using 17314 SNP markers and on an additional panel of 152 breeding lines (76 % tolerant and 24 % susceptible) using in particular internal (non-public) chlorotoluron phenotyping data, as well as 62 000 Exome SNP variants on chromosome 6B (focusing on gene coding area), all physically anchored on Chinese Spring.

The GWAS analysis with the 379 lines allowed to fine-map the Su1 area within 0.2 cM (internal genetic consensus map) and 5.847 Mb on Chinese Spring assembly between markers cfn0876173 and cfn0877589 at position chromosome 6B 157778805 and 163625566 respectively. The complementary GWAS analysis with the panel of 152 lines allowed to define a confidence interval of 15.796 Mb, between position chromosome 6B 157778805 and 173574762. Despite this new interval was larger due to the small size of the panel and the accuracy of the phenotyping data, it was overlapping with the previous one and confirmed the left border at position chromosome 6B 157778805.

Computing the Exome sequence reads mapping coverage by gene, a small genomic area without any mapping coverage was identified, containing 3 predicted genes on Chinese Spring IWGSC annotation V1.1 (TraesCS6B02G158300, TraesCS6B02G158400 and TraesCS6B02G158500). The absence of reads mapping coverage which could come from a deletion event was highly correlated with the chlorotoluron susceptible phenotype. It was observed that 82% of the susceptible lines of the panel were deleted in this area while none of the tolerant lines showed the deleted pattern.

The larger confidence interval, chr6B 157778805 to 173574762, was containing 119 high confidence genes and 214 Low confidence genes, based on Chinese Spring IWGSC annotation V1.1.

SEQ ID NO: 3 was identified as being the Su1 causal gene. This causal gene, TraesCS6B02G158300 located on Chinese Spring assembly at position chr6B 160899142 to 160902898 is composed of 2 exons and can be characterized as a CYP71C4 protein, a cytochrome P450 monooxygenase (CYP) classified as a A-type family (CYP71) from cluster C4.

### Example 2. Analysis of wheat CYP71C4 gene

### Activity assays in yeast

The cDNA coding for the CYP71C4 sequence from chlorotoluron tolerant wheat (SEQ ID NO: 2 followed by nucleic acid encoding for several histidines (His-Tag) was cloned via GATEWAY LR recombination in a yeast expression vector. Expression of the CYP71C4 enzyme in yeast and assays of chlorotoluron conversion with yeast microsomes was performed as described by Abdollahi *et al.* (2021). Microsomes from transformed yeast cells were incubated for 1 hr at 27°C in the presence of 200 µM chlorotoluron. The converted chlorotoluron product was then quantified via a high-performance liquid chromatography (HPLC) analysis as described by Abdollahi *et al.* (2021). This product was then identified as a hydroxylated derivative of chlorotoluron (hydroxylation of the methyl group present on the chlorotoluron carbon ring) via LC-MS/MS analysis (Figure 1).

### Cloning of wheat CYP71C4 gene for chlorotoluron tolerance

The cDNA CYP71C4 sequence from a chlorotoluron tolerant wheat (SEQ ID NO: 2) was cloned via a Golden Gate reaction into the destination binary plasmid pBIOS10746, between the constitutive rice actin 1 promoter (proOsAct1) with the rice actin 1 first intron (intOsAct1) (McElroy et al., 1990) and a 3' termination sequence of the gene encoding a sorghum heat shock protein (accession number: Sb03g006880).

The binary destination vector pBIOS13790 is a derivative of the binary vector pMRT (WO2001018192A3). The final binary plasmid was transformed into Agrobacterium EHA105.

### Knock-down of CYP71C4 gene via CRISP/Cas9 genome editing in wheat tolerant cultivar

A CRISPR/cas9 approach was undertaken to knock-down the functional CYP71C4 gene present on chromosome 6B of chlorotoluron tolerant Fielder wheat cultivar. To this purpose, two SpCas9 gRNAs targeting the CYP71C4 gene at position 86 (CCGGGCAACAAGCACGAGAC, SEQ ID NO: 9) and 363 (AGGGCGTCGACGAGCGCGTT, SEQ ID NO: 10) of the CYP71C4 coding region, driven each by the wheat TaU6 RNA Pollll promoter were cloned via a Golden Gate reaction into the destination binary plasmid pBIOS11974 harboring the SpCas9 gene flanked with Sv40 NLS and Nucleoplasmin NLS sequences under the control of the constitutive maize Ubiquitin promoter (proZmUbi) with the maize ubiquitin intron (intZmUbi, Christensen et al. 1992) and a 3' termination sequence of the gene encoding an *Agrobacterium tumefaciens* Nopaline Synthase terminator (An et al., 1985). The final binary plasmid, named pBIOS13769, was transformed into Agrobacterium EHA105.

### Introducing a functional CYP71C4 gene in wheat susceptible cultivar

### Preparation of the vector

The cDNA corresponding to the functional CYP71 gene from cultivar ARINALRFOR was cloned under the control of the rice Actin1 promoter and first intron and a 16.9 kDa sorghum Heat Shock Protein terminator (Sb03g006880) via a Golden Gate reaction into a plant binary vector containing a plant transformation selectable marker. The final binary plasmid, named pBIOS13790, was transformed into Agrobacterium EHA105.

### Wheat Transformation

Fielder (chlorotoluron tolerant) or Cadenza (chlorotoluron susceptible) wheat cultivars were transformed with these agrobacterium strains essentially as described by WO2000/063398. Wheat transgenic events were generated for each construct described above. The Fielder cultivar was transformed with construct pBIOS13769 whereas the Cadenza cultivar was transformed with construct pBIOS13790. All wheat transgenic plants were grown in a glasshouse under standard wheat growth conditions (16 hr of light period at 20°C and 8 hr of dark period at 15°C with constant 60% humidity.

### Molecular characterization of CRISPR/cas9 edited plants and phenotypic characterization

Fielder plants transformed with pBIOS13769 were analyzed for the presence of T-DNA and mutations within its CYP71C4 gene. To analyze for the presence of mutations two PCR fragments (one for each gRNA targeted site) were amplified with primer pairs PP_04257 and PP_004275 and sequenced to confirm Cas9 edition.

**Table 2. Primers for determining presence of mutation.**

| | | | |
|---|---|---|---|
| PP_04257 _F | 5’- GTCGTGGTCAGAAGATC ACAG-3’, SEQ ID NO: 11 | PP_04257 _R | 5'- GATGAGAGGGAGCTTG TTCG-3', SEQ ID NO: 12 |
| PP_04275 _F | 5’- CTCATGCTCCTCCGCCT C-3’, SEQ ID NO: 13 | PP_04275 _R | 5'- CGCCAGAACTCGCCGT AG-3', SEQ ID NO: 14 |

### Mutated plants were kept and grown in the glasshouse.

To assay for chlorotoluron tolerance or susceptibility, T1 plants (progeny of transformed wheat plants) are grown in the glasshouse until the growth stage BBCH13 (3 developed leaves) and sprayed with a solution of chlorotoluron (Shvat herbicide) at a concentration of 3 g/L and a spraying rate equivalent to what is used by farmers (600 L/ha).

### Use of the CYP71C4 locus as a landing pad

### Introduction downstream (3') of the CYP71C4 encoding gene

A functional Gene of Interest (GOI) expression cassette can be inserted downstream of the CYP71C4 locus of a chlorotoluron susceptible line (figure 2). To this purpose, the non-functional CYP71C4 gene is opened by the CRISP-Cas9 system with 2 gRNAs, one being targeted upstream of the GGACCTACG sequence (Cleavage 1 gRNA) and the second one after the CYP71C4 terminator sequence (Cleavage 2 gRNA), in order to remove the non-functional version of the CYP71C4 gene (creating a partially deleted version of the CYP71C4gene). In order to insert the GOI expression cassette and to reconstitute a functional CYP71C4 gene, the fragment to be inserted within the partially deleted CYP71C4 locus contains at least 200 nucleotides of the CYP71C4 gene upstream the Cleavage 1 gRNA site and a region of at least 200 nucleotides downstream of the Cleavage 2 gRNA targeted site.

To avoid cleavage of the repair fragment containing the CYP71C4 fragment with the corrected GGACCACG
sequence and the GOI expression cassette, the 2 gRNA recognition sites on the repair sequence have to be mutated (mutation represented by the * in the figure) in such a way that there is no recognition of repair fragment by the gRNA. This mutation is defined such a way to keep the same amino acid translated sequence for Cleavage site 1.).

### Introduction upstream (5') of the CYP71C4 encoding gene

A functional Gene of Interest (GOI) expression cassette can be inserted upstream of the CYP71C4 locus of a chlorotoluron susceptible line (figure 3). To this purpose, the non-functional CYP71C4 gene is opened by the CRISP-Cas9 system with 2 gRNAs, one being targeted in the genomic region upstream of the CYP71C4 gene promoter and the second one downstream of the GGACCTACG sequence in order to remove the non-functional version of the CYP71C4 gene. In order to insert the GOI expression cassette and to reconstitute a functional CYP71C4 gene, the fragment to be inserted within the partially deleted CYP71C4 locus contains at least 200 nucleotides... of genomic sequence upstream of the Cleavage site 1 region (upstream of the CYP71C4 gene promoter) and a region of at least 200 nucleotides... downstream of the Cleavage 2 gRNA targeted site located downstream of the GGACCTACG sequence. To avoid cleavage of the repair fragment containing the CYP71C4 fragment with the corrected GGACCACG sequence and the GOI expression cassette, the 2 gRNA recognition sites on the repair sequence have to be mutated (mutation represented by the * in the name) in such a way that there is no recognition of the repair sequence by the gRNA. This mutation is defined such a way to keep the same amino acid translated sequence for Cleavage site 2).

## Claims

1. A method for making a cell resistant to chlorotoluron, comprising expressing SEQ ID NO: 1 within the cell.

2. The method of claim 1, wherein expression of SEQ ID NO: 1 is achieved by introducing a nucleic acid coding for SEQ ID NO: 1 in the genome of the cell.

3. The method of claim 1 or 2, wherein the cell contains defective copies of SEQ ID NO: 3 and a gene coding for SEQ ID NO: 1 is obtained by gene editing of at least one native defective copy of SEQ ID NO: 3 in the genome of the cell.

4. The method of claim 1 or 2, wherein the gene coding for SEQ ID NO: 1 is introduced in the genome of the cell by transgenesis.

5. A method for performing genetic transformation of cells, comprising
a) Transforming cells with a vector comprising a nucleic sequence allowing production of SEQ ID NO: 1, wherein the cells are susceptible to chlorotoluron,
b) Growing the transformed cells on a medium containing chlorotoluron
c) Selecting the cells able to grow on the medium of b).

6. The method of claim 5, wherein the nucleic sequence allowing production of a functional gene coding for SEQ ID NO: 1 is SEQ ID NO: 2.

7. The method of claim 5 or 6, wherein the vector further comprises at least one other sequence of interest coding for another protein imparting a phenotypical trait of interest.

8. The method of any one of claims 5 to 7, wherein the nucleic sequence allowing production of SEQ ID NO: 1, and optionally at least one other sequence of interest coding for another protein imparting a phenotypical trait of interest, are introduced within the genome of the cell by homologous recombination.

9. Use of SEQ ID NO: 1 as a selective marker for cell transformation, in particular of plant cells.

10. A method for making a transgenic plant, comprising introducing a nucleic acid sequence coding for SEQ ID NO: 1 at the UBI locus in the cells of the plant, wherein SEQ ID NO: 1 is in frame with the UBI gene, so that a fusion protein UBI-SEQ ID NO: 1 is produced.

11. A method for determining whether a plant is susceptible to chlorotoluron, comprising searching for the presence of a mutation in the gene coding for SEQ ID NO: 1, wherein the mutation leads to formation of a truncated protein or to absence of production of the protein, wherein a mutation is present on both alleles of the plant, in particular wherein the mutation is an insertion of "T" after position 787 of SEQ ID NO: 3.

12. A method for determining whether a plant is resistant to chlorotoluron, comprising determining presence of one functional allele of the gene coding of the protein depicted by SEQ ID NO: 1, wherein presence of one functional allele in the genome of the plant is indicative of the plant resistance to chlorotoluron.

13. The method of any one of claims 11 to 12, which is performed by sequencing of the locus of SEQ ID NO: 3, amplification of a region located in SEQ ID NO: 3 or use of molecular markers.

14. A method for increasing resistance to chlorotoluron in a plant, comprising modifying the promoter region of the gene coding for SEQ ID NO: 1, wherein expression of SEQ ID NO: 1 is enhanced as compared to a plant in which the enhancers have not been introduced and wherein the plant is resistant to higher amounts/concentrations of chlorotoluron.

15. A transgenic cell, or a transgenic plant comprising a gene coding for SEQ ID NO: 1, as a transgene.

16. A method for making a plant sensitive to chlorotoluron, comprising inhibiting the expression of the gene coding for the CYP71C4 protein represented by SEQ ID NO: 1 in said plant.
